(19) Europäisches Patentamt
European Patent Office
Office européen des brevets

(11) **EP 1 660 133 B1**

(12) **EUROPEAN PATENT SPECIFICATION**

(45) Date of publication and mention of the grant of the patent:
**18.04.2018 Bulletin 2018/16**

(21) Application number: **04733284.6**

(22) Date of filing: **17.05.2004**

(51) Int Cl.:
*A61K 47/64* (2017.01) *A61K 31/7056* (2006.01)
*A61P 31/12* (2006.01)

(86) International application number:
**PCT/CA2004/000741**

(87) International publication number:
**WO 2004/100995 (25.11.2004 Gazette 2004/48)**

(54) **TARGETED DELIVERY OF ANTI-VIRAL COMPOUNDS THROUGH HEMOGLOBIN BIOCONJUGATION**

GEZIELTE ABGABE VON ANTIVIRALMITTELN DURCH HÄMOGLOBIN-KONJUGIERUNG

ADMINISTRATION CIBLÉE DE COMPOSÉS ANTIVIRAUX AU MOYEN DE BIOCONJUGUÉS A BASE D'HÉMOGLOBINE

(84) Designated Contracting States:
**AT BE BG CH CY CZ DE DK EE ES FI FR GB GR HU IE IT LI LU MC NL PL PT RO SE SI SK TR**

(30) Priority: **15.05.2003 US 470445 P**
**24.10.2003 US 513575 P**

(43) Date of publication of application:
**31.05.2006 Bulletin 2006/22**

(73) Proprietor: **Therapure Biopharma Inc.**
**Mississauga ON L5N 8H9 (CA)**

(72) Inventors:
- **ADAMSON, J., Gordon**
**Georgetown, Ontario L7G 1V3 (CA)**
- **BELL, David**
**Oakville, Ontario L6H 4A7 (CA)**
- **NG, Nancy**
**Vaughan, Ontario L4K 2E1 (CA)**
- **BIESSELS, Peter**
**Toronto, Ontario M6S 3H9 (CA)**

(74) Representative: **D Young & Co LLP**
**120 Holborn**
**London EC1N 2DY (GB)**

(56) References cited:
**EP-A- 1 367 393** **WO-A-92/13875**
**WO-A-94/11399** **WO-A-95/05397**
**WO-A-99/56723** **WO-A-03/100419**
**US-A- 5 439 882**

- **ZUWALA-JAGIELLO J ET AL: "INTERNALIZATION STUDY USING EDTA-PREPARED HEPATOCYTES FOR RECEPTOR-MEDIATED ENDOCYTOSIS OF HAEMOGLOBIN-HAPTOGLOBIN COMPLEX" INTERNATIONAL JOURNAL OF BIOCHEMISTRY AND CELL BIOLOGY, EXETER, GB, vol. 30, no. 8, August 1998 (1998-08), pages 923-931, XP001075085 ISSN: 1357-2725 cited in the application**
- **KLUGER RONALD ET AL: "Bioconjugation of homogeneous cross-linked hemoglobin by chemical design" ARTIFICIAL CELLS, BLOOD SUBSTITUTES, AND IMMOBILIZATION BIOTECHNOLOGY, MARCEL DEKKER INC, US, vol. 22, no. 5, November 1994 (1994-11), page A115, XP002110925 ISSN: 1073-1199**
- **CHAMOW S M ET AL: "Conjugation of Soluble CD4 without Loss of Biological Activity via a Novel Carbohydrate-directed Cross-linking Reagent" JOURNAL OF BIOLOGICAL CHEMISTRY, AMERICAN SOCIETY OF BIOLOGICAL CHEMISTS, BALTIMORE, MD, US, vol. 267, no. 22, 5 August 1992 (1992-08-05), pages 15916-15922, XP002267698 ISSN: 0021-9258**
- **ANTONINI E ET AL: "THE OXYGEN EQUILIBRIUM OF HUMAN HEMOGLOBIN CONJUGATED WITH FLUORESCEIN ISOTHIOCYANATE" BIOCHIMICA ET BIOPHYSICA ACTA, AMSTERDAM, NL, vol. 2, no. 82, 1964, pages 355-360, XP001077800 ISSN: 0006-3002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 660 133 B1

- KRISTIANSEN M ET AL: "Identification of the haemoglobin scavenger receptor" NATURE, MACMILLAN JOURNALS LTD. LONDON, GB, vol. 409, no. 6817, 2001, pages 198-201, XP002215001 ISSN: 0028-0836 cited in the application
- MOLLER H J ET AL: "Identification of the hemoglobin scavenger receptor/CD163 as a natural soluble protein in plasma" BLOOD, vol. 99, no. 1, 1 January 2002 (2002-01-01), pages 378-380, XP002297216 ISSN: 0006-4971
- LEVY GARY A ET AL: "Ribavirin coupled to hemoglobin protects against viral hepatitis induced by the coronavirus MHV-3 in mice." HEPATOLOGY, vol. 38, no. 4 Suppl. 1, October 2003 (2003-10), page 305A, ABSTRACT NO. 301, XP008035303 & 54TH ANNUAL MEETING OF THE AMERICAN ASSOCIATION FOR THE STUDY OF LIVER DISEASES; BOSTON, MA, USA; OCTOBER 24-28, 2003 ISSN: 0270-9139
- LEBLEBICIOGLU H ET AL: "Treatment of acute hepatitis C virus infection with interferon-[alpha] 2b and ribavirin: Case report and review of the literature" ANNALS OF CLINICAL MICROBIOLOGY AND ANTIMICROBIALS 14 OCT 2002 UNITED KINGDOM, [Online] vol. 1, 14 October 2002 (2002-10-14), page 3p, XP002297217 ISSN: 1476-0711 Retrieved from the Internet: URL:http://www.ann-clinmicrob.com/content/ 1/1/3>
- POL S ET AL: "Hepatitis C and human immune deficiency coinfection at the era of highly active antiretroviral therapy" JOURNAL OF VIRAL HEPATITIS 2002 UNITED KINGDOM, vol. 9, no. 1, 2002, pages 1-8, XP002297218 ISSN: 1352-0504
- ZEIN C O ET AL: "Advances in therapy for hepatitis C infection" MICROBES AND INFECTION 2002 FRANCE, vol. 4, no. 12, 2002, pages 1237-1246, XP002297219 ISSN: 1286-4579
- LUXON B A ET AL: "Pegylated interferons for the treatment of chronic hepatitis C infection" CLINICAL THERAPEUTICS 01 SEP 2002 UNITED STATES, vol. 24, no. 9, 1 September 2002 (2002-09-01), pages 1363-1383, XP002297220 ISSN: 0149-2918
- BROWN T J ET AL: "Antiviral agents: Nonantiviral drugs" JOURNAL OF THE AMERICAN ACADEMY OF DERMATOLOGY 01 OCT 2002 UNITED STATES, vol. 47, no. 4, 1 October 2002 (2002-10-01), pages 581-599, XP002297221 ISSN: 0190-9622
- RITTER M ET AL: "Interaction of CD163 with the regulatory subunit of casein kinase II (CKII) and dependence of CD163 signaling on CKII and protein kinase C" EUROPEAN JOURNAL OF IMMUNOLOGY, WEINHEIM, DE, vol. 31, no. 4, April 2001 (2001-04), pages 999-1009, XP002255899 ISSN: 0014-2980
- WILLEMS M ET AL: "Liver transplantation and hepatitis C" TRANSPLANT INTERNATIONAL 2002 GERMANY, vol. 15, no. 2-3, 2002, pages 61-72, XP002297222 ISSN: 0934-0874
- HEUVEL VAN DEN M M ET AL: "REGULATION OF CD163 ON HUMAN MACROPHAGES: CROSS-LINKING OF CD163 INDUCES SIGNALING AND ACTIVATION", JOURNAL OF LEUKOCYTE BIOLOGY, vol. 66, no. 5, November 1999 (1999-11), pages 858-866, XP002943525, ISSN: 0741-5400

**Description**

## FIELD OF THE INVENTION

This invention relates to the field of targeted drug delivery. In another aspect the invention relates to hemoglobin-ribavirin compound conjugates for use in treatment of viral and inflammatory conditions wherein the conjugate directs delivery of the ribavirin to a macrophage.

## BACKGROUND OF THE INVENTION

### Targeted Drug Delivery

The importance of targeted drug delivery to enhance treatment regimes is well known in the art. Drug formulations (e. g. drug carriers used, pill, vs. liquid form, pill coatings) and mode of delivery (e. g. intravenous, oral, inhalation) can have an impact on the effectiveness of a drug and its side effects. However, optimization of a treatment regime using targeted drug delivery is a challenge. A number of factors need to be taken into account: knowledge of the condition to be treated, mechanism of action of a drug, identification of a target site for the drug and a suitable carrier or formulation to enable the delivery of drugs to the target site in a manner wherein the drug retains its activity.

One targeted drug delivery technique developed in recent years is receptor-mediated delivery. This has the advantage of high specificity of delivery to the cells which express a receptor for the drug carrier (ligand).

The specific targeting of low molecular weight therapeutic and diagnostic agents to tissues is enhanced greatly through the use of receptor-mediated delivery. Diagnostic agents such as fluorescent or radiolabeled substances can be used to indicate the location and quantity of cells bearing the targeted receptors when such agents are administered as complexes with ligands for those receptors. These complexes are also useful in characterizing the binding and transport properties of receptors on cells in culture. Such information is useful in detection of, and/or design of therapy for, tissues containing the target cells, either in vitro or in vivo. However, it is still a challenge to identify optimal targets and effective modes of delivery to desired targets.

Certain targeted drug delivery means have been identified. For instance, means of drug delivery to macrophages that have previously been described, include, e. g., liposomes, gold, gold-labeled liposomes, polystyrene or carbon particles, macrophage-specific antibodies, microspheres, nanoparticles, lipoproteins, erythrocytes, and pathogens known to infect macrophages. (See for example, United States Patent Nos. 6,599,887; 6,448,932; 6,096,311;6,071,517; 6,018,031 and 4,764,359 ; and Schmidt, J. et al., Brain (2003) 126 (8): 1895-904). In another embodiment, carriers modified by polyethylene glycol (PEG), amphiphiles, peptides or proteins such as fibronectin, tuftsin, gelatin, or glycosylated carriers may also be used. However, all of said delivery methods have limitations. For instance, there can be leakage of drugs with use of liposomes or microspheres, as well as issues related to, solubility, heterogeneity of size, biodistribution, and toxicity, biocompatibility with artificial components and cost.

### Hemoglobin

Hemoglobin, as a natural component of red blood cells, is present and circulating throughout the body in relatively large quantities. Hemoglobin has well-established bioacceptability and clearance mechanisms, and the potential to transport drugs through the circulatory system.

Thus, Kluger et al., U. S. Pat. No. 5,399, 671 and WO 94/11399 describe a hemoglobin compound which has been cross-linked to effect intramolecular stabilization of the tetrameric structure thereof, but which contains a residual functional group on the cross-linker residue to which drugs for delivery can be covalently attached.

Anderson et al., U. S. Pat. No. 5,679, 777, describes complexes of hemoglobin compounds and polypeptide drugs, in which the polypeptide drug is bound to a globin chain through a disulfide linkage to a cysteine unit inherent in, or genetically engineered into, the globin chain.

Haptoglobins (Hp) constitute part of the $\alpha_2$-globin family of serum glycoproteins. Haptoglobins are present in mammalian plasma, and constitute about one-quarter of the $\alpha_2$-globulin fraction of human plasma. Each individual has one of three phenotypic forms of haptoglobin, of close structural and chemical identity. Haptoglobins are composed of multiple $\alpha\beta$ dimers and the phenotypes are conventionally denoted Hp 1-1, Hp 2-1 and Hp 2-2. The $\beta$ chains are identical in all haptoglobin phenotypes, but the $\alpha$ chains vary ($\alpha^1$ and $\alpha^2$). The amino acid sequences of all chains are known. Hp 1-1 is composed of two $\alpha^1\beta$ dimers and has a molecular weight of about 98 kDa. The structure of Hp 2-1 and Hp 2-2 can be written as follows: $(\alpha^1\beta)_2$ $(\alpha^2\beta)_n$ where n=0,1, 2,... and $(\alpha^2\beta)_m$ where m=3,4,5,... respectively.

One function of haptoglobin is to bind extracellular hemoglobin, arising from red blood cell lysis, to form essentially irreversible haptoglobin-hemoglobin complexes that are recognized by specific receptors. Hemoglobin-haptoglobin receptors have been identified on hepatocytes in the liver and more recently on macrophages. In this way, hemoglobin

is targeted to the liver or macrophages for metabolism. Further, CD163 has also been identified as a hemoglobin-haptoglobin receptor on macrophages (Kristiansen et al, Identification of the haemoglobin scavenger receptor. Nature 409,198-201 (2001)). In one aspect, hemoglobin-haptoglobin can be targeted to cells containing CD163 on their cell surface, such as macrophages.

## Anti-viral Therapy

Currently, there are limited options in the treatment of viral conditions. Because viruses incorporate into the infrastructure of the host cell, developing drugs that are specific or have a sufficient specificity to viral infected cells with minimum toxicity to non-infected host cells is a challenge. A therapeutic index which is minimum toxicity dose to a host cell divided by the minimum effective dose that is toxic to a virus that favours the use of the anti-viral is desirable. One example of a suitable range for the therapeutic index is 100-1000, but this invention is not bound by such a range.

Many viruses encode for their own RNA/DNA polymerases or other proteins or enzymes necessary for their replication or function, such as proteases, mRNA capping enzymes, neuramidases, ribonucleases, and kinases. Samples of such viruses include, but are not necessarily limited to Hep B, Poz, Irido, herpes, Adeno, Corona, Rhabdo, Paramyxo, Orthomyxo, Toja, Reo nand Picorna viruses. Anti-viral therapy often targets nucleic acid synthesis. This can be affected in a number of ways, for instance, where viral polymerases are more sensitive to the drug than the host enzymes. Thymidine kinase is one enzyme that is encoded by some viruses, and can activate drug to toxic form, wherein uninfected cells do not. So some anti-virals can be administered in pro-drug form and designed to be activated in cells comprising said viral thymidine kinase. Administering drugs in a non-phosphorylated form, can make it easier for the drug to enter a cell, whose membrane is poorly permeable to phosphorylated drugs. The drug can then be phosphoylated to an active form by the thymidine kinase. Alternatively, one could administer the drug in an active phosphorylated form, where mode of delivery permits, in which case the drug would be active without further processing.

One class of anti-viral agents that has been used is nucleoside analogues. Nucleoside analogues have an altered sugar, base or both. Examples of nucleoside analogues include idoxuridine, acyclovir (acycloguansoine), ganiciclovir, adensosine arabinoside (AraA, Vidarabine), Ara-AMP, AraC (cytarabine), Ara-CMP, azidothymidine (AZT), ribavirin, didenosine (DDI), dideoxycytosine (DDC), stavudine (d4T), Epivir (3TC), abacavir (ABC), iodo-deozyuridine (DU), Valacyclovir, and bromovinyl deoxiuridine (BVDU).

Nucleoside analogs that include sugar modifications are acyclovir (a guanosine analogue), ganiciclovir (a 2'-deoxyguanosine analogue, similar to acyclovir but with an extra hydroymethyl group on its side chain), Valacyclovir is the hydrochloride salt of l-valyl ester of acyclovir, AraA, DDI, DDC. Nucleoside analogues with base modifications include DU, BVDU, and Ribavirin which is a guanosine analogue.

Most of the anti-viral nucleoside analogues target nucleic acid synthesis, or thymidine kinase. Resistance or viral mutations to overcome the therapies can develop. Further, delivery of an effective amount in a suitable time period to a desired site while minimizing side effects has been a challenge in anti-viral therapy. Administered alone, the uptake of these class of anti-virals tends to at least some degree be non-specific and are associated with a number of toxic side effects including hemolytic anemia.

Nucleoside analogues of guanosine have been developed and include acyclovir, ganiciclovir, Valacyclovir and ribavirin. One such guanosine analogue is ribvairin. Cyclic guanosine analogs have been found to be useful substrates for varicella-zoster (AR Karlstrom, et al., Antimicrob Agents Chemother. 1986 January, 29(1):171-174). They have also been shown to be effective against cytomegalavirus (CMV) (B. Watinen, A. Larsson, U. Ruden, A. Sundquist, E. Solver, 1987 February, 31(2):317-320).

## Ribavirin

Ribavirin is a known nucleoside analogue and is used in anti-viral treatment against a wide range of RNA and DNA viruses. It is an analogue of guanosine (Hoffman et al, 1973, Antimicrob. Agents Chemother. 3: 235; Sidwell et al, 1972, Science 177: 205) that was developed by ICN Pharmaceuticals Inc. It is approved for the treatment of infections caused by RSV (respiratory syncitial virus) and HCV (hepatitis C virus). Current combination therapy to treat HCV involves use of interferon-alpha (IFNoc) and ribavirin. Ribavirin is also one of the only drugs that was used in the treatment for SARS (severe acute respiratory syndrome). Ribavirin by itself is ineffective as an anti-viral agent in the treatment of HCV infection, but combined with IFNA, increases the rate of sustained viral response. However, it takes 4 weeks of dosing to achieve steady state plasma levels of ribavirin and ribavirin is taken up non-specifically by all body tissues. As such, current treatments require daily administration of high doses (800-1200 mg/day) for periods of 24-48 weeks. This is not practical for treatment of non-chronic conditions, such as acute disease like SARS and the like. Further, current treatment of HCV infections using ribavirin is limited by ribavirin toxicity. The most frequent side effect of ribavirin is the development of hemolytic anemia, which occurs in-10% of patients. Generally, hemoglobin levels decrease by 3 g/dL or more in 54% of all patients. The percentage of patients who achieve a sustained viral response using pegylated

IFN$\alpha$ and ribavirin is at best 50-60%. The ribavirin toxicity can result in either dose reduction or its discontinuation in certain patients, with a consequent reduction in response to therapy. The mechanism for the beneficial action of ribavirin is not entirely understood, as ribavirin appears not to eradicate viral replication.

As such there is a need for improved anti-viral nucleoside analogue treatment and therapy. There is also a need for improved delivery and targeted delivery of said anti-viral nucleoside analogues, wherein ribavirin is the anti-viral drug, and for an improved toxicity profile.

WO 99/56723 describes hemoglobin-haptoglobin construct complexes to which a hepatocyte-modifying agent, such as ribavirin, is attached. The construct-complex may be formed *in vivo* or ex *vivo.*

WO 92/18375 and US 5,439,882 describe a blood substitute comprising purified hemoglobin, preferably bovine, cross-linked intramolecularly with periodate-oxidized ATP (o-ATP) and intermolecularly with periodate-oxidized adenosine (o-adenosine), combined with reduced glutathione (GSH), and optionally enriched with mannitol, non-electrolytes, and/or electrolytes.

WO 95/05397 describes hemoglobin-based red cell substitutes featuring stable nitroxide free radicals for use in cell-free hemoglobin solutions, encapsulated hemoglobin solutions, stabilized hemoglobin solutions, polymerized hemoglobin solutions, conjugated hemoglobin solutions, nitroxide-labelled albumin, and nitroxide-labelled immunoglobulin. The formulations described therein interact with free radicals, act as antioxidant enzyme-mimics, and alleviate oxidative stress and oxygen-related toxicity.

EP1367393A and WO 03/100419, published after the earliest priority date of the present application, describe the identification of a molecule capable of interacting with a soluble and/or cell bound form of CD163 and as a result of said interaction an immune response is either instigated or suppressed in an organism, as well as to pharmaceutical compositions comprising them and their use in the treatment of immune response disorders.

R. Kluger et al. Artificial Cells, Blood Substitutes, and Immobilization Biotechnology, 1994, 22(5), A115, describes the bioconjugation of homogeneous cross-linked hemoglobin by chemical design.

S.M. Chamow et al, Journal of Biological Chemistry, 1992, 267 (22), 15916-15922, describes conjugation of soluble CD4 without loss of biological activity via a novel carbohydrate-directed cross-linking reagent.

E. Antonini et al, Biochimica Biophysica Acta, 1964, 2 (82), 355-360, describes the oxygen equilibrium of human hemoglobin conjugated with fluorescein isothiocyanate.

H.J. Møller et al, Blood 2002 99:378-380; describes the hemoglobin scavenger receptor (HbSR/CD163) as an interleukin-6- and glucocorticoid-regulated macrophage/monocyte receptor for uptake of haptoglobin-hemoglobin complexes.

G. Levy et al, Hepatology, 2003, 38(4) Suppl. 1, 305A, published after the earliest priority date of the present application, describes conjugation of ribavirin to haemoglobin and its protective effect against viral hepatitis induced by the coronavirus MHV-3 in mice.

H. Leblebicioglu et al. Annals of Clinical Microbiology and Antimicrobials, 2002, 1, 3p, describe treatment of acute hepatitis C virus infection with interferon-a 2b and ribavirin.

S. Pol et al., Journal of Viral Hepatitis 2002, 9(1), 1 8, reviews the potential impact of highly active antiretroviral therapies (HAART) on hepatitis C as well as the interactions between HIV and HCV therapies.

C.O. Zein et al, Microbes and Infection 2002, 4(12), 1237-1246, reviews therapies for hepatitis C infection.

B.A. Luxon et al, Clinical Therapeutics 2002, 24(9), 1363-1383, describes pegylated interferons and their use in the treatment of chronic hepatitis C infection.

T.J. Brown et al. J Am Acad Dermatol 2002, 47, 581-599, is a review of antiviral agents discussing the treatments available for viral infections such as herpes simplex virus, varicella zoster virus, cytomegalovirus, human papillomavirus, chronic viral hepatitis, and others.

M. Ritter et al, European Journal of Immunology, 2001, 31(4), 999 - 1009, describes the interaction of CD163 with the regulatory subunit of casein kinase II (CKII) and dependence of CD163 signalling on CKII and protein kinase.

M. Willems et al, Transplant International 2002, 15(2-3), 61 - 72, relates to hepatitis C infection before and after liver transplantation.

## SUMMARY OF THE INVENTION

In one aspect, the present invention provides a hemoglobin-nucleoside analogue conjugate for use in treating an inflammatory or viral condition, where the conjugate directs delivery of the nucleoside analogue to a macrophage, wherein the nucleoside analogue is ribavirin.

In a preferred embodiment, the hemoglobin is a mammalian hemoglobin, more preferably bovine or human hemoglobin, most preferably human hemoglobin. In another embodiment, the hemoglobin can be isolated and purified from red blood cells or from cell culture. In yet another embodiment, the hemoglobin can be recombinant hemoglobin. In another embodiment, the hemoglobin may be of natural sequence or a variant thereof, including truncated or composite sequences.

The hemoglobin is conjugated to the ribavirin, directly or indirectly through a linker. In yet another embodiment, the hemoglobin is non-intramolecularly cross-linked. In one embodiment, the hemoglobin is non-intra and non-intermolecularly crosslinked. In another embodiment, the hemoglobin drug conjugate is capable of binding haptoglobin. In another embodiment, the drug is conjugated to the hemoglobin at a site independent of the haptoglobin binding site. The drug may also be conjugated to the haptoglobin portion of the hemoglobin-haptoglobin complex.

In one embodiment the invention provides hemoglobin-ribavirin conjugates for use in the treatment of viral infections, such as coronavirus, RSV, HCV or the like. The invention also provides said conjugates for use in the treatment of viral hepatitis.

In another aspect the invention provides a pharmaceutical composition comprising a conjugate of hemoglobin and ribavirin for use in the treatment of a viral infection wherein said conjugate directs delivery of said ribavirin to macrophages.

In one embodiment, the viral infection is selected from the group consisting of MHV-3, Hepatitis C, HIV, SARS, RSV, coronavirus.

In another embodiment, the invention provides a hemoglovin-ribavirin conjugate for use in treating a condition that is modulated through macrophages (i. e. modulation of inflammatory and immune responses),
wherein said conjugate directs delivery of the drug to macrophages. In a further embodiment, the agent is capable of modulating the immune response or the agent is capable of modulating the inflammatory response of the macrophage. In more specific embodiments, macrophage function is modulated through the secretion of specific cytokines such as IFN, such as IFN$\gamma$, or TNF$\alpha$, or through the expression of certain surface molecules such as CD163, MHC class II and associated co-stimulatory and adhesion molecules.

Other features and advantages of the present invention will become apparent from the following detailed description.

## BRIEF DESCRIPTION OF THE DRAWINGS

The invention will now be described in relation to the drawings in which:

**Figure 1** illustrates the method used to prepare hemoglobin ribavirin conjugates of the invention in one embodiment of the invention.

**Figure 2** Anion-exchange HPLC of Hb-ribavirin conjugate using a pH gradient (pH 8.5-6. 5) under non-denaturing conditions.

**Figure 3** is a MALDI-TOF mass spectrometry profile of a Hb-ribavirin conjugate. The increase in MW of each of the a and globin chains by multiples of the ribavirin phosphate mass (308 Da) confirms addition of multiple drugs per globin chain.

**Figure 4** illustrates biological activity of ribavirin enzymatically cleaved from Hb-ribavirin conjugate. Human HepG2 (**left-A**) and mouse AM12 (**right-B**) hepatic cells were treated with ribavirin (□), and ribavirin cleaved and purified from Hb-ribavirin conjugate (■). The cells were assayed for uptake of bromodeoxyuridine (BrdU) as a measure of cell proliferation. Inhibition of cell proliferation is represented as % inhibition of control untreated cells.

**Figure 5** illustrates *in vitro* uptake of Hb-ribavirin conjugate by hepatic and non-hepatic cells. Human hepatoma HepG2, mouse AM12 hepatocyte, and 5637 bladder carcinoma cells were assayed for internalization of fluorescein-labeled Hb and Hb-ribavirin conjugate at 37°C for 2 hr. Results are expressed as fold increase in relative fluorescence units (RFU) over equivalent samples not treated at 37°C.

**Figure 6** is a bar graph illustrating the clinical behaviour of MHV-3 infected mice by plotting the composite clinical score versus days post infection for PBS, haptoglobin-hemoglobin-ribavirin conjugate (HRC 203) and ribavirin alone treated mice. Scores range from zero (dead) and 1 (poor) to 5 (normal).

**Figure 7** is a micrograph illustrating the histopathogy results for day 3 livers in PBS control, hemoglobin-ribavirin and ribavirin treated, MHV-3 infected mice. It illustrates the degree of liver necrosis and fibrin deposition after infection with MHV-3 in the three treatment regimes.

**Figure 8** is a bar graph illustrating the survival of MHV-3 coronavirus-infected mice under the three treatement regimes: (1) Control (PBS); (2) haptoglobin-hemoglobin-ribavirin (HRC203); and (3) ribavirin.

**Figure 9** is a bar graph illustrating the mean viral titer in livers of MHV-3 infected mice (PFU/gm versus days post-infection) for the three treatment regimes: (1) Control (PBS); (2) haptoglobin-hemoglobin-ribavirin (HRC203); and (3) ribavirin.

**Figure 10** is a bar graph illustrating the viral titer in macrophages infected in vitro with MHV-3 under the three treatment regimes: (1) Control (PBS); (2) haptoglobin-hemoglobin-ribavirin (HRC203); and (3) ribavirin.

**Figure 11** is a bar graph illustrating the percent inhibition of viral replication in macrophages infected in vitro with MHV-3 under the three treatment regimes: (1) Control (PBS); (2) haptoglobin-hemoglobin-ribavirin (HRC203); and (3) ribavirin.

**Figure 12** is a bar graph illustrating that hemoglobin-ribavirin conjugate decreases cytokine production *in vitro* in macrophages infected with MHV-3: (A) TNF$\alpha$ and (B) IFN$\gamma$.

## DETAILED DESCRIPTION

In one aspect, the present invention provides a hemoglobin-nucleoside analogue conjugate for use in the treatment of an inflammatory of viral condition, said conjugate directing delivery of the nucleoside analogue to a macrophage, wherein the nucleoside analogue is ribavirin. In another aspect, said conjugate is capable of binding to or is bound to haptoglobin.

The present inventors have shown that not only can the delivery of such hemoglobin-ribavirin conjugates be targeted to cells comprising a hemoglobin or a hemoglobin-haptoglobin receptor, they can also improve the efficacy and safety of said ribavirin over the use of said ribavirin alone. The present invention enables the use of a reduced dose of ribavirin as compared to a control, such as the use of the ribavirin alone, in the treatment of a viral condition.

In one aspect, the present inventors have also shown that such hemoglobin-ribavirin conjugates can be directed to cells comprising a hemoglobin receptor, such as a hemoglobin-haptoglobin receptor.

In another aspect the hemoglobin-ribavirin conjugates of the present invention, in use, decrease the macrophage mediated immune response, such as that associated with TNF-$\alpha$ or IFN, such as IFN-$\gamma$, expression.

In one aspect the conjugate is capable of binding to haptoglobin or is further bound to haptoglobin to form a haptoglobin-hemoglobin-anti-viral nucleoside analogue complex. Said haptoglobin can be directly or indirectly (e.g., through a linker) to said hemoglobin. In one aspect said complex is formed in a way that permits binding of the complex to a hemoglobin-haptoglobin receptor.

In one embodiment, the hemoglobin is conjugated to the anti-viral nucleoside analogue, either directly or through a linker. The hemoglobin can be non-intramolecularly cross-linked, or cross-linked hemoglobin (intra-and/or inter-molecularly cross-linked). In another embodiment, the hemoglobin is human hemoglobin.

The anti-viral nucleoside of the conjugate is ribavirin. The conjugate and composition used in the present invention can be used to treat a viral infection, or inflammatory conditions, such as autoimmune disorders where immunosuppression or suppression of TNF-$\alpha$ or IFN, such as IFN-$\gamma$, is desired.

It should be noted that the hemoglobin-ribavirin conjugates used in the present invention can be used alone or in the preparation of a medicament for the treatment of medical conditions as noted herein, and/or in combination therapies. For instance, the hemoglobin-ribavirin conjugates used in the present invention can be used in combination with IFN, such as IFN-$\gamma$, therapy.

### Hemoglobin

The hemoglobin compound useful as a component of the conjugates used in the present invention can be substantially any hemoglobin compound providing the necessary degree of biocompatibility for administration to a patient or animal, the necessary sites for attachment of the drug or other substance of interest, and preferably having sufficient binding affinity for haptoglobin. Within these limitations, it can be a naturally occurring hemoglobin from human or animal sources. It can be a non-intramolecularly cross-linked hemoglobin. It can be a modified natural hemoglobin, e. g. an intramolecularly cross-linked form of hemoglobin to minimize its dissociation into dimers, an oligomerized (intra-and/or non-intra-molecularly cross-linked oligomers) form or a polymerized form. It can be a hemoglobin derived from recombinant sources and techniques, with its naturally occurring globin chains or such chains mutated in minor ways. It can be comprised of subunits or fragments of Hb, or derivatives thereof, which have affinity for haptoglobin. It can be a hemoglobin in which individual amino acids of the globin chains have been removed or replaced by site specific mutagenesis or other means. In one embodiment of the invention, certain modifications which are known to decrease the affinity of hemoglobin for binding to haptoglobin are in one embodiment of the invention, preferably avoided in hemoglobin compounds used in the present invention. Modifications to hemoglobin and/or haptoglobin that enhance haptoglobin

binding to hemoglobin or the conjugate of the invention or binding of the conjugate or complexes used in the invention to a cell or particular cell type are encompassed.

One type of hemoglobin compounds are those which comprise hemoglobin tetramers intramolecularly cross-linked to prevent their dissociation into dimers, and which leave functional groups available for chemical reaction with the drug or other substance, either directly or through a chemical linker molecule. Such hemoglobin compounds provide a known, controlled number of reactive sites specific for the therapeutic substance of interest, so that an accurately controlled quantity of the therapeutic substance can be attached to a given amount of hemoglobin compound. They also avoid utilizing sites on the globin chains for linkage to the therapeutically active substance drug or other substance, so as to minimize conformation disruption of the globin chains and minimize interference with the hemoglobin-haptoglobin binding and with binding of the construct-complex to the receptor.

Human hemoglobin, e. g. that is obtained from outdated red blood cells, and purified to a desired level, such as by the displacement chromatography process described in U.S.Pat. No. 5,439,591 Pliura et al. is one raw material that can be used for preparation of the hemoglobin product for use in the conjugate and/or complex used in the present invention. In one embodiment, this material may be cross- linked with a trifunctional cross-linking agent as described in aforementioned U.S.Pat. No. 5,399,671, Kluger et al., namely a reagent which utilizes two of its functional groups for intramolecular cross-linking between subunits of the hemoglobin tetramer, and leaves its third functional group available for subsequent reaction with a nucleophile. A specific example of such a cross-linking reagent is trimesoyl tris (3,5-dibromosalicylate), TTDS, the preparation of which is described in the aforementioned Kluger et al. U.S.Pat. No. 5,399,671.

The ribavirin can be bound to the hemoglobin, either directly or through a chemical linker or spacer, and then this complex may be administered to the patient so that the haptoglobin-hemoglobin binding takes place in vivo. The entire haptoglobin-hemoglobin-drug complex can, if desired, be formed extracorporeally and then administered to the patient, and this can under some circumstances lead to better control of the amounts of active substance finally being delivered to the hemoglobin or hemoglobin-haptoglobin receptor bearing macrophages. However, such a procedure is not normally necessary, save for those exceptional patients having zero or low levels of haptoglobin, e.g. in conditions of acute hemolysis. Such patients can be administered haptoglobin before, during and/or after administration of the hemoglobin-ribavirin conjugate of the invention. Usually, however, there is sufficient haptoglobin in the patient's plasma to form the haptoglobin-hemoglobin-ribavirin complex *in situ* to effect its delivery to the target cells. Preparation of the hemoglobin-ribavirin and administration of that to the patient, to form the haptoglobin-hemoglobin-ribavirin complex *in situ* is generally cheaper and less complicated.

Use of intramolecularly crosslinked hemoglobins will give rise to high molecular weight polymers containing more than one hemoglobin and/or haptoglobin owing to the presence of multiple binding sites on each of these proteins. There may be advantages to using non-crosslinked hemoglobin as a component of the conjugates and complexes used in present invention. Such a hemoglobin, with a drug or other substance bound to it, will dissociate into dimeric hemoglobin of approximate molecular weight 32 kDa, and two such dissociated dimeric hemoglobin products bind to a single molecule of haptoglobin to give a complex according to the present invention. The formation of high molecular weight haptoglobin-hemoglobin complexes is thus avoided. Haptoglobin binding to $\alpha\beta$-dimers is generally a much faster reaction than haptoglobin binding to crosslinked hemoglobin. The lower molecular weight complexes resulting from the use of non-crosslinked hemoglobin may show improved receptor binding and uptake.

Where hemoglobin of a form which will dissociate into dimers is used as a component of the present invention, or where hemoglobin dimers themselves are used, for example, where the dimers have been modified such that they cannot reform 64 kDa hemoglobin, the haptoglobin-hemoglobin-ribavirin conjugate-complex can be formed according to the invention extracorporeally, and then the finished conjugate-complex is administered to the patient, so as to avoid the risks attendant on administering to the body a molecular species of too small a molecular weight, namely, clearing the drug too rapidly through excretion. Administration of Hb dimers bearing ribavirin may be possible without prior binding to haptoglobin in cases where complex formation in vivo is adequate prior to clearance of the modified dimer.

A further example of a hemoglobin compound useful in conjugate-complexes and conjugates used in the present invention is dimeric hemoglobin bearing a modifying group containing thiol, preferably a terminal side chain thiol, of the type described in U. S. Provisional Patent Application of Kluger and Li, entitled "Hemoglobin With Chemically Introduced Disulfide Crosslinks and Preparation Thereof", filed Nov. 3, 1997. Appropriate chemistries can be used for attachment of ribavirin to such dimeric hemoglobin, either by direct reaction with the exposed thiol, or by direct reaction with an activated form of the thiol, or by mixed disulfide formation, or through a linker molecule. Conjugate- complexes of this type are made extracorporeally and administered to a patient in this form. The ribavirin conjugate can also be administered for *in vivo* Hp binding. The use of dissociable hemoglobin (32 kDa molecular weight) has the advantage over the use of cross-linked hemoglobin tetramers in that they provide an exposed dimer-dimer interface which facilitates haptoglobin binding.

The conjugate-complexes and conjugates used in the present invention may also utilize hemoglobin which has been modified in a manner which results in impaired nitric oxide binding. Such modified hemoglobins are known in the

art. Reduced NO binding may reduce the tendency of the hemoglobin to effect modifications to a patient's blood pressure upon administration, an effect which has been noted with some hemoglobins, even in small amounts.

In forming the conjugate-complex or conjugate, it may be necessary to interpose between the reactive site on the hemoglobin chosen and the ribavirin, a chemical linker or a spacer group. This depends upon the nature of the available chemical group on hemoglobin for linking, and on the chemical groups available on the substance to be bound to hemoglobin, for this purpose. For example, ribavirin may be conjugated at one of its ribose hydroxyl groups to an amino group of the hemoglobin through a phosphoramidate linkage. Such a linkage may be cleaved enzymatically or otherwise in the target cell and liberate the phosphorylated form of the nucleoside analogue in the cell.

In one embodiment, there may be advantages to using polymerized Hb, such as increased circulation time whether pre-complexed with Hp or not, and in the case of no pre-complexation, then the increased circulation time would possibly allow for more complete complexation in vivo. The larger polyHb-Hp complexes may have altered recognition and uptake by macrophages, also. Both intramolecularly and non-intramolecularly polymerized hemoglobin can be used.

With regard to the formation of hemoglobin-haptoglobin ribavirin conjugates, used in the present invention, such conjugates can be made in accordance with the method disclosed in United States patent number 6,479, 637. A conjugate-complex according to one embodiment of the present invention comprises a haptoglobin molecule, which may be haptoglobin 1-1 or any other phenotype, bonded to one or more molecules of a hemoglobin compound by means of strong non-covalent interaction. The hemoglobin may be cross-linked, oligomerized or unmodified, as described above.

For instance, in one embodiment, the hemoglobin-haptoglobin conjugate-complex or conjugate according to the present invention can be generally represented by the formula:

$$(Hp)_a - (Hb)_b - (L_c - A_d)_e$$

where

a=1 to about 10;
b=0.5 to about 10;
c=0 to about 10;
d=1 to about 20;
e=1 to about 20;
Hp is haptoglobin as described herein;
Hb is a hemoglobin as described herein; in one embodiment, it is a non-intramolecularly cross-linked Hb;
L is a linker as described herein; and
A is ribavirin, or pharmaceutically acceptable salts thereof,

in which the stoichiometry of Hp to Hb in the complex is dictated by the available number of binding sites on the two proteins, but is generally of the order of 1:0.5 to 1:2.

In yet another embodiment, the hemoglobin can be oxyhemoglobin, deoxyhemoglobin, carboxyhemoglobin, or met-hemoglobin.

Other forms of hemoglobin or compounds that can deliver drugs to cells comprising hemoglobin-haptoglobin receptors can also be used.

## Receptors/Carriers

The hemoglobin-haptoglobin complexes, whether formed in vivo or ex vivo are known to bind to receptors on hepatocytes. Not wishing to be bound by any particular theory or mechanism of action, CD163 receptors, (one form of hemoglobin-haptoglobin receptor) are present on macrophages and. can bind hemoglobin-haptoglobin complexes. CD163 is disclosed in Kristiansen et al., "Identification of the haemoglobin scavenger receptor" (2001) Nature 409: 198-201, published on Jan. 11, 2001 as binding specifically to Hb-Hp, and that this receptor is found on macrophages and certain monocytes. Activation of the CD163 receptor triggers a cascade of intracellular events leading to an anti-inflammatory response. As such the present invention provides hemoglobin conjugated to ribavirin for use in treating inflammatory or viral conditions, wherein the conjugate can deliver the ribavirin to macrophages.

Further, the conjugate-complexes or conjugates used in the present invention may exert beneficial effects on neighboring cells, if the ribavirin that is bound to the hemoglobin is, for example, one which is active towards neighboring cells even if they are not cells having receptors for the hemoglobin-ribavirin conjugates or complexes used in the present invention.

In general, when a hemoglobin-ribavirin conjugate is used, in one embodiment, several molecules of the ribavirin are attached to each hemoglobin tetramer, in a manner that still enables haptoglobin binding. If non-intramolecuarly

cross-linked, the hemoglobin dissociates into 2 dimers and is tightly bound by the plasma protein haptoglobin. The haptoglobin-hemoglobin-ribavirin complex is carried through the bloodstream to cells bearing receptors for the complex and is internalized through an endocytic pathway. Once inside the cell, the ribavirin is enzymatically or otherwise cleaved from the complex and released into the cell cytoplasm to exert its biological effect.

In one embodiment of the invention, the hemoglobin-ribavirin is a hemoglobin-phosphoramidate-ribavirin, for instance, such as described in the examples. In this form, the ribavirin may be cleaved from the hemoglobin in phosphorylated form. As such, the ribavirin is released into the cell cytoplasm in an active form or at least in a form that does not require further phosphorylation for its activity.

Hemoglobin is used as the drug delivery agent in the examples below, with unexpected enhanced effects.

The conjugates and complexes used in the present invention can be used in conjunction with other therapies. For instance, hemoglobin-ribavirin conjugates can be used in conjugation with IFN therapy or treatment.

## Anti-viral Nucleoside Analogues

Nucleoside analogues have an altered sugar, base or both.

The nucleoside analogue used in the conjugates for use in the present invention is ribavirin which is a guanosine analogue.

The nucleoside analogue that is used in the conjugates used in the present invention is well known in the art and is ribavirin or a pharmaceutically acceptable salt thereof.

In one aspect the analogues of the present invention are capable of binding to hemoglobin either directly or through a linker.

## Ribavirin

Ribavirin is a nucleoside analogue and has been used in anti-viral therapy and as an immunomodulator. The present inventors are the first to determine that delivery of ribavirin directly to macrophages enhances its activity. They were also the first to deliver ribavirin to hemoglobin-haptoglobin receptor bearing cells, such as hepatocytes, and to CD163 receptor bearing cells, such as macrophages, and have shown that this unexpectedly enhances the effectiveness of ribavirin therapy. As such the present invention provides hemoglobin-ribavirin conjugates, and ribavirin compositions, for use in treating an inflammatory or viral condition wherein the conjugate directs delivery of the ribavirin to a macrophage.

Ribavirin that can be used in the present invention can be ribavirin as disclosed in Figure 1 or shown below (I) or a pharmaceutically acceptable salt thereof.

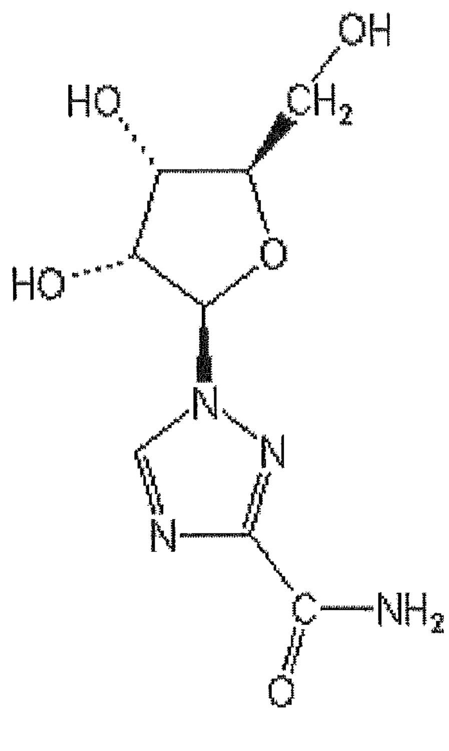

The use of the term "ribavirin" herein includes ribavirin, or pharmaceutical acceptable salts thereof. All of these

can be used in the invention as long as they are capable of conjugating to hemoglobin. In one embodiment, they bind to hemoglobin (directly or indirectly through a linker or the like), in a manner that enables the conjugate to bind to haptoglobin.

## Hemoglobin-Ribavirin Conjugates

The present invention provides a hemoglobin-ribavirin conjugate for use in the treatment of an inflammatory or viral condition, wherein the conjugate directs delivery of the nucleoside to macrophages. Selective uptake of haptoglobin-hemoglobin-ribavirin has been herein demonstrated in vitro in hepatic cells and cells expressing CD163, and enhanced effect of haptoglobin-hemoglobin-ribavirin vs. free ribavirin has been demonstrated in vitro in macrophages and in vivo in virus-infected mice. In another embodiment, the invention also provides a hemoglobin-ribavirin conjugate for use in the treatment of an inflammatory or viral condition, wherein the conjugate is capable of binding haptoglobin or that comprises haptoglobin. It avoids the systemic toxicity associated with chronic nucleoside therapy such as ribavirin therapy. The conjugate of the present invention can achieve greater efficacy of ribavirin therapy. With regard to hemoglobin-ribavirin conjugates, it also is effective in maintaining optimal ribavirin levels in patients, such as HCV patients, who would otherwise require dose reduction or discontinuation of ribavirin therapy. Acid phosphatase, a lysosomal enzyme, has been shown to release bioactive ribavirin from hemoglobin-ribavirin in vitro. Similar release of active drug is expected following lysosomal uptake of the conjugate in target cells.

The hemoglobin-ribavirin conjugate, can in one embodiment be formed via the reaction scheme shown in Figure 1 or outlined in Example 2. A person skilled in the art would appreciate that other modes of conjugation could be used. In another embodiment, the hemoglobin can be any hemoglobin as previously described herein. In another embodiment, the ribavirin can be a ribavirin or a pharmaceutically acceptable salt of ribavirin as previously described herein.

In one embodiment, the present invention provides hemoglobin-ribavirin analogues that are hemoglobin phosphoramidate ribavirin. The bond may enable cleavage of the nucleoside analogue in a phosphorylated form. In one embodiment this is the active form of the ribavirin.

In one embodiment, the molar ratio of Hb: ribavirin is 1:5 - 1:20. In one embodiment the molar ratio is 1:5 - 1:15.

In one embodiment, the molar ratio of Hb:ribavirin is about 1:5 to about 1:10. In another embodiment, the molar ratio is about 1:8 (Hb:ribavirin).

## Pharmaceutical Compositions

Diclosed herein are pharmaceutical compositions comprising the hemoglobin-ribavirin for use in the present invention. In one aspect the pharmaceutical compositions can target delivery of ribavirin to macrophages. Pharmaceutical compositions that can deliver the ribavirin to hemoglobin or hemoglobin-haptoglobin receptor bearing cells are also disclosed. The compositions may be administered to living organisms including humans, and animals.

The pharmaceutical composition may be administered in a convenient manner such as by direct application to the infected site, e. g. by injection (subcutaneous, intravenous, etc.). In case of respiratory infections, such as SARS, it may be desirable to administer the conjugates, such as the ribavirin compositions used in the present invention directly to the lungs, through known techniques in the art. Depending on the route of administration (e. g. injection, oral or inhalation, although injection is a preferred mode of administration), the pharmaceutical compositions may be coated in a material to protect the compound from the action of enzymes, acids and other natural conditions that may inactivate the compound.

The compositions described herein can be prepared by *per se* known methods for the preparation of pharmaceutical acceptable compositions which can be administered to subjects, such that an effective quantity of the ribavirin is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985) or Handbook of Pharmaceutical Additives (compiled by Michael and Irene Ash, Gower Publishing Limited, Aldershot, England (1995)). On this basis, the compositions include, albeit not exclusively, solutions of the substances in association with one or more pharmaceutical acceptable vehicles or diluents, and may be contained in buffered solutions with a suitable pH and/or be iso-osmotic with physiological fluids. In this regard, reference can be made to U. S. Patent No. 5,843, 456. As will also be appreciated by those skilled, administration of substances described herein may be by an inactive viral carrier.

## Applications

Administration of a therapeutically effective amount, "effective amount" or "sufficient amount" of pharmaceutical compositions used in the present invention is defined as an amount effective, at dosages and for periods of time necessary to achieve the desired result, including clinical results, and, as such, an "effective amount" depends upon the context

in which it is being applied. For example, a therapeutical active amount of a substance may vary according to factors such as the disease state, age, sex, and weight of the individual, mode and form of administration and the ability of the substance to elicit a desired response in the individual. Dosage regimes may be adjusted to provide the optimum therapeutic response. For example, several divided doses may be administered daily or the dose may be proportionally reduced as indicated by the exigencies of the therapeutic situation. For example, in the context of administering an effective amount of ribavirin, ribavirin conjugate or hemoglobin- ribavirin conjugate of the present invention is an amount sufficient to achieve such desired activity; e. g. anti-viral, and/or macrophage modulator; and/or cytokine modulator; and/or immunomodulator; and/or inflammatory response modulator.

In one embodiment, the effective amount is based on plasma concentration of the nucleoside. In another embodiment it is based on dosage per day or dosage per kg of body weight. The desired amount can depend on desired use or mode of administration.

As used herein, and as well understood in the art, "treatment" is an approach for obtaining beneficial or desired results, including clinical results. Beneficial or desired clinical results can include, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

The term "modulate" as used herein includes the inhibition or suppression of a function or activity (such as ribavirin activity or macrophage activity) as well as the enhancement of a function or activity.

The term "animal" as used herein includes all members of the animal kingdom including human that can benefit from treatment with the drug conjugates used in the present invention, for instance animals that have or are carriers of viral infections, such as birds, mammals (such as horses, pigs, dogs, cats, humans). In one embodiment, the animal is a human.

The phrase, "Subject in need thereof" as used herein means any animal that has or is suspected of having a condition that can be treated or prevented by the hemoglobin-ribavirin of the present invention.

The hemoglobin-ribavirin conjugates are used in the present invention to target macrophages to modulate the immune and inflammatory response, preferably to enhance the anti-viral and/or immunomodulatory and/or anti-inflammatory activity of ribavirin by targeting them to macrophages. The conjugates used in the present invention also increase the efficacy of ribavirin due to increased ribavirin analogue bioavailability, half-life and stability. It also reduces systemic toxicity by targeting ribavirin analogues to macrophages. In one embodiment, the ribavirin analogues can be delivered using carriers known to target and deliver drugs and other substances to macrophages, including analogues or derivatives of hemoglobin that are capable of binding haptoglobin and that bind the hemoglobin or hemoglobin-haptoglobin receptors. The ribavirin analogue can be conjugated to hemoglobin or to hemogobin derivatives in a manner that enables binding to a suitable receptor, such as CD163, said receptor being known to be present on macrophages and hepatocytes and can target ribavirin-like compound delivery to these sites. The ribavirin conjugates used in the present invention can be used to control or treat conditions related to immune/inflammatory response, or other responses mediated by macrophages.

The hemoglobin-ribavirin conjugates and/or complexes are used in the present invention in the treatment of inflammatory or viral conditions. It can enhance the treatment of viral infections. For instance, ribavirin compositions, conjugates and complexes of the present invention can be used in the treatment of coronaviruses (e. g. , MHV-3, SARS), hepatitis C, RSV, Lassa Fever and the like, by administration to a subject in need thereof.

The present invention shall now be illustrated by the following examples. Such examples are for illustrative purposes only.

## EXAMPLES

### Example 1 - Preparation of Hemoglobin

Stroma free hemoglobin was prepared using techniques known in the art. In the present instance, human hemoglobin was obtained from outdated red blood cells, and purified by the displacement chromatography process described in United States patent Number 5,439,591 (Pliura et al.). Non-intramolecularly cross-linked hemoglobin was used for the Examples below.

### Example 2 - Preparation of Hemoglobin-Ribavirin Conjugate

**Synthesis of Ribavirin Phosphate Imidazolide.**

Ribavirin phosphate was synthesized by derivatisation of ribavirin at its primary hydroxyl group using phosphooxychloride and dimethylphosphate (Allen, et al., J Med Chem. 1978 Aug;21(8):742-6.), and monitored for ribavirin modification by C18 reverse-phase HPLC. Following completion of the reaction, the ribavirin phosphate (1 mmol) was mixed with 10 g of fine charcoal (100-400 mesh). The charcoal-reaction mixture was centrifuged at 2000 g for 15 min and the supernatant recovered. The wash steps were repeated until no inorganic phosphate could be detected in the supernatant as assayed using the Ames method (Ames BN (1966), Assay of inorganic phosphate, total phosphate and phosphatases. Methods Enzymol 8: 115-118). The charcoal was extracted with EtOH/water/$NH_4OH$ (10: 10: 1) and the pooled extract evaporated to dryness. The resulting ribavirin phosphate ammonium salt was converted to its free acid (Streeter et al, Proc. Natl. Acad. Sci. USA 1973 Apr; 70 (4): 1174-8). Purity of the ribavirin phosphate was evaluated using 2 assays. Acid phosphatase was used for complete enzymatic cleavage of ribavirin from ribavirin phosphate, followed by quantification of the released ribavirin by C18 reverse-phase HPLC. C18 reverse-phase HPLC was performed on a C18 Phenomenex Luna column (4.6x250) using isocratic elution with water/TFA 0.1% pH 2.9, flow 1 ml/min. Total inorganic phosphate content of ribavirin phosphate was measured using the Ames method (described below). Purified ribavirin phosphate was converted to its imidazolide (Fiume et al., Anal Biochem. 1993 Aug 1:212(2):407-11), with slight modifications. The reaction was performed under dry $N_2$, using dry solvents. Typically, 324 mg (1 mmol) of ribavirin phosphate was dissolved in 10 ml anhydrous DMF. 5 mmol carbonyldiimidazole in 5 ml DMF was added, followed by 5 mmol imidazole in 5 ml DMF. The solution was stirred at RT for 30 min and the DMF evaporated. The remaining oil/solid was dissolved in 2 ml anhydrous EtOH, followed by precipitation with the addition of 20 ml anhydrous ether. The precipitate was washed twice with ether, and residual ether was removed with a gentle stream of dry $N_2$. The ribavirin phosphate imidazolide was used immediately for conjugation to Hb.

**Conjugation Of Ribavirin Phosphate Imidazolide With Hb.**

0.06 μmol of Hb (CO form) was mixed with 6.6 μmol ribavirin phosphate imidazolide at a final concentration of 0.8 μM in 0.1 M $NaHCO_3/Na_2CO_3$, pH 9.5. The pH of the reaction mixture was monitored over the first hr, and maintained at pH 9.5 - 9.6 by addition of 0.2 M $Na_2CO_3$. After the pH of the reaction had stabilized, the reaction mixture was charged with CO for 15 min and the reaction allowed to continue under CO at 37°C for 96 hr. Hb was monitored for drug modification using anion-exchange chromatography. Anion exchange chromatography was performed on a Poros H/HQ (4.6/100) column, using a pH gradient 8.3-6.3 over 10 min (mobile phase 25 mM Tris pH 8.3, 25 mM bisTris pH 6.3) with a flow rate of 4 ml/min. All Hb was modified as evidenced by later elution on anion exchange media relative to unmodified Hb control, due to the added net negative charge resulting from modification of lysine side chain amino groups with the phosphate containing conjugant (**Figure 2**). The conjugate was dialysed (MWCO 10 kDa) against Ringer's Lactate (3X 0.5 L), sterile filtered (0.2 μm) and charged with CO prior to storage at - 80°C.

**Determination Of Molar Drug Ratio.**

The molar drug ratio of Hb-ribavirin conjugate was determined by quantification of ribavirin released by enzymatic cleavage using the acid phosphatase assay and determination of total inorganic phosphate using the inorganic phosphate assay. The molar concentration of Hb protein was determined using the Drabkins assay kit for Hb (Sigma). MALDI-TOF mass spectrometric analysis of the Hb-ribavirin conjugate indicated up to at least 5 ribavirin phosphate groups attached to both alpha and beta chains of the Hb (**Figure 3**). However, on average 8 ribavirin molecules covalently linked to each hemoglobin molecule. For the acid phosphatase assay, 5 nmol (0.3 mg) of the Hb-ribavirin conjugate was diluted into 0.3 ml of 1 mM NaOAc/HAc buffer, pH 4.8. 3 units of a freshly prepared acid phosphatase (Type IV-S, potato) was added, and the enzymatic reaction allowed to proceed at 37°C for 2 hr. Hb precipitate was removed by centrifugation and the supernatant analysed for ribavirin by C18 reverse-phase HPLC. For evaluation of biological activity of released ribavirin, the supernatant was dialysed against PBS and concentrated prior to analysis. For the inorganic phosphate assay, total inorganic phosphate was determined using the method of Ames, 1966. The volume of 10% $Mg(NO_3)_2$ in EtOH was optimized to 80 μl for assay of 5 nmol Hb-ribavirin conjugate.

**Hp Binding Assay.**

To determine retention of Hp binding by Hb-ribavirin conjugate, a complex was allowed to form with a 10% molar excess of human Hp, at RT for a minimum of 30 min. Size exclusion HPLC (SEC) analysis indicated formation of a higher MW complex corresponding to the Hp complex of the Hb-ribavirin. SEC was performed using a Pharmacia Superdex 200 column using 0.5M MgCl2/25 mM Tris, pH 7.2 at a flow rate of 0.4 ml/min, with detection at 414 nm. Formation of the complex of conjugate with Hp was confirmed by elution of Hb-containing species that appeared as peaks eluting earlier than the non-complexed Hb-drug conjugates.

**Preparation Of The Fluorescein-Hb-Ribavirin Double Conjugate.**

A 1 mM solution of fluorescein maleimide (Pierce 46130) was prepared in PBS. Hemoglobin or hemoglobin-ribavirin conjugate was added to a concentration of 100 μM, and incubated 4 hr in the dark at RT with gentle agitation. The sample was then dialyzed extensively against PBS to remove any unbound fluor. Based on the concentration of fluor and protein in the purified conjugate, determined by fluorimetry and Coomassie analysis, respectively, the ratio of fluor label to Hb-RV was approximately 1. RP HPLC analysis coupled with fluorescence detection showed all fluorescence to be associated with the β-chain, indicating the expected attachment of the fluorescein maleimide to the surface reactive βCys93 thiol group. Binding of the fluorescein labelled Hb-RV to Hp was also verified by size exlusion HPLC analysis. Binding to Hp was verified the a shifting of the Hb derivative peaks to an earlier retention time corresponding to an increase in molecular weight upon formation of the Hp-Hb(Fl)-RV complex.

***In vitro* Bioactivity Assay of Ribavirin From Hb-Ribavirin Conjugate.**

Ribavirin recovered from acid phosphatase cleavage of Hb-ribavirin conjugate was evaluated for bioactivity in an *in vitro* cell proliferation assay using the Cell Proliferation ELISA Bromodeoxyuridine (BrdU) kit (Roche, Cat. No. 1 647 229). Human hepatoma HepG2 cells and mouse hepatocyte AML12 cells were plated at a density of 4 x $10^4$ cells/well, and 1 x $10^4$ cells/well, respectively, in flat bottom 96-well plates. The cells were allowed to grow for 24 hours, at which time they were treated in quadruplicate with ribavirin or ribavirin from Hb-ribavirin conjugate for 6 hours. The treatments were removed, and fresh media containing BrdU was added to the wells and the incubation continued for 18 hr. The standard BrdU ELISA assay was then followed according to the kit protocol. Cleaved ribavirin activity was equivalent to unmodified ribavirin control **(Figure 4),** demonstrating the ribavirin is not detrimentally altered by the conjugation and cleavage processes, and suggesting that activity of ribavirin cleaved from the conjugate *in vivo* will have activity similar to free ribavirin.

**Internalization Assay.**

To evaluate uptake of Hb-ribavirin conjugate by hepatic cells, internalization assays were performed using fluorescein-tagged Hb and Hb-ribavirin conjugate (Zuwala-Jagiello and Osada, 1998, "Internalization study using EDTA-prepared hepotocytes for receptor-mediated endocytosis of haemoglobin-haptoglobin complex"; The International Journal of Biochemistry & Cell Biology; England, Aug. 1998, vol. 30, No. 8; pp. 923-931, XP00107508).

HepG2 cells and 5637 bladder carcinoma cells were plated in 12-well plates at 2.5 x $10^6$ cells/well, and mouse AM12 hepatocytes at 2 x $10^5$cells/well. The cells were allowed to grow for 48 hours. Media was removed and the cells were washed with HBSS containing 2 mg/ml BSA (HBSS/BSA). Hb or Hb-ribavirin conjugate labelled with fluorescein was complexed with Hp (1:1 molar ratio) in HBSS/BSA, and added to cells to a final concentration of 500 μg/ml. The labelled complexes were allowed to bind for 2 hr at 4°C. ATP was added to 1 mM and receptor-mediated internalization was initiated by incubation at 37°C for various times. The cells were washed with HBSS/BSA and surface-bound ligands were stripped from cells by incubation in 0.2 M acetic acid/0.5 M NaCl for 10 minutes. The cells were washed with PBS and lysed with 2 M NaOH. The solubilized cell extract was transferred to a flat bottom 96 well plate, and fluorescence measured (485 nm excitation/530 nm emission) using a fluorometric plate reader (Packard Fluorocount). Both the Hb and Hb-ribavirin, complexed to Hp, were taken up by the liver derived cell lines (HepG2 and mouse AM12) and neither was effectively internalized by the non-liver cell line (5637 bladder carcinoma), demonstrating the selective targeting of the Hb-ribavirin complex to cells bearing receptors the Hb-Hp complex (**Figure 5**). A labelled albumin control was not significantly internalized by any of the cell lines, thereby confirming that the level of Hb uptake in the liver cell lines was not due to passive transport of macromolecules.

**Example 3 - *In vitro* and *In vivo* Studies Of Hemoglobin-Ribavirin Conjugates In The Treatment of MHV-3**

The drug delivery effects of free ribavirin and hemoglobin-ribavirin conjugate (Hb-ribavirin), prepared as in Example 2 and complexed to haptoglobin, were compared in mice infected with murine hepatitis virus strain 3 (MHV-3), a coronavirus that produces fulminant hepatitis in mice. The molar ratio of conjugated ribavirin to hemoglobin was approximately 8:1.

**Methods.**

These studies were designed to examine the potential for haptoglobin-hemoglobin-ribavirin (Hp-Hb-Ribavirin) to protect against MHV-3 infection *in vivo* and to assess the anti-viral and anti-inflammatory effects in cultures of macrophages *in vitro.*

*In vivo*

| | |
|---|---|
| Day -1 | Treatment (All infusions were 100 μl in PBS)<br>1) PBS (n=5)<br>2) Hp-Hb-Ribavirin (6 mg RV/kg/ay, n=10)<br>3) Ribavirin (18 mg RV/kg/day, n=10) |
| Day 0 | Infection (i.p. 100 pfu MHV-3 in PBS)<br>+ Treatment (1, 2, 3) |
| Days 2-5 | Daily:<br>Measure survival<br>Sacrifice 2 mice per group for measures of:<br>- Serum ALT<br>- Hemoglobin<br>- Hematocrit<br>- Liver viral titre<br>- Liver histopathology<br><br>+ Treatment of remainder (1, 2, 3), excluding Day 5 (end of study) |

Mice were divided into 3 treatment groups as follows :

Group 1: Mice infected with MHV-3 and treated with PBS as controls (n=5)
Group 2: Mice infected with MHV-3 and treated with Hp-Hb-ribavirin at 6 mg conjugated ribavirin/kg/day (n=10)
Group 3: Mice infected with MHV-3 and treated with ribavirin at 18 mg/kg/day (n=10)

All groups of mice were infected with 100 plaque forming units (PFU) of MHV-3 by intraperitoneal injection. Treatments were given daily by intravenous tail vein injection starting at day-1 and continuing to the end of the experiment. Blood samples were collected daily, prior to daily test article infusion, and analyzed for evidence of hepatitis by liver biochemistry (alanine aminotransferase, bilirubin) disturbances in hematologic parameters (hemoglobin, white blood cell count, platelet count), renal dysfunction (creatinine, blood urea nitrogen). Liver tissues were collected, fixed in formalin and examined by routine histology (hematoxylin and eosin) for hepatic necrosis and by immunohistochemistry for fibrin deposition and necrosis. Viral titers were determined by cytopathic effect assay using snap frozen liver tissue.

*In Vitro*

Macrophages were isolated from mice after injection of intraperitoneal thioglycollate. Macrophages were pretreated with free ribavirin (200 μg/ml) or Hp-Hb-ribavirin (1 mg/ml containing approximately 10 μg conjugated ribavirin/ml) one hr prior to infection with 1000 PFU MHV-3 (m.o.i. 10-3). At intervals, macrophages were harvested and analyzed for viral titers and production of inflammatory mediators, tumor necrosis factor-α (TNF-α) and interferon-y (IFN-y).

**Results.**

Clinical Behaviour

Mice treated with the hemoglobin-ribavirin conjugate of the invention exhibited superior clinical behaviour after infection with MHV-3. The infected controls (PBS/MHV-3 alone) at day 2-4 had ruffled fur, were shaking and were inactive. The infected mice treated with ribavirin alone had ruffled fur and were lethargic at day 2-5. However, the infected mice treated with the hemoglobin-ribavirin conjugate of the invention exhibited superior clinical behaviour (i.e., were active, had normal respiration and fur texture and no shaking) and behaved like uninfected normal mice. A graph of the composite clinical score versus days post-infection for the three test groups is illustrated in **Figure 6.**

Anemia

MHV-3 infected mice treated with hemoglobin-ribavirin do not develop anemia that is normally caused by ribavirin therapy. As stated previously, prior art reports that ribavirin therapy causes does-limiting hemolytic anemia. In the present study, the ribavirin group showed decreased hematocrit and hemoglobin below the normal range consistent with anemia over the 3 days following infection, while the hemoglobin-ribavirin treated animals stayed in the normal range (See **Table 1**). On day 5 it was noted that the ribavirin treated animal were dehydrated as compared to the hemoglobin-ribavirin treated animals which results in elevating their hematocrit and hemoglobin levels.

Liver Necrosis and Fibrin Deposition

Histopathology results indicated that treatment of MHV-3 infected mice with Hp-Hb-Ribavirin resulted in delaying and reducing the course of liver necrosis and fibrin deposition caused by MHV-3 infection as compared to untreated controls. Reduction in liver necrosis was similar between the Hp-Hb-Ribavirin and free ribavirin groups, despite the fact that the dose of conjugated ribavirin in the Hp-Hb-Ribavirin was only 1/3 of that of free ribavirin used. The results at day 3 are illustrated in **Figure 7** and described below.

*Day 3 Liver: PBS Control (Figure* 7 e, *f) (350X magnification)*

There are marked diffuse hepatic cellular changes with multiple areas of confluent hepatocellular necrosis throughout the liver. Approximately 60% of the liver is necrotic. The immunostain shows striking fibrin deposits that match the areas of necrosis; the fibrin is deposited especially in sinusoids within and around the areas of necrosis. This is the classical hepatic pathology of MHV-3 induced murine fulminant viral hepatitis. The natural progression is rapid extension of the necrosis to involve the entire liver once it has reached this stage. It is graded here as 3+ out of 4.

*Day 3 Liver: Hp-Hb-Ribavirin-treated group (Figure 7 g, h) (150X magnification)*

The liver is characterized by widely scattered microfoci of liver cell necrosis. The lesions are very discreet. The immunostain shows sharp localization of fibrin in sinusoids in the areas of necrosis. This is early hepatic necrosis in MHV-3 viral hepatitis. Hepatic changes are variable. The extent of the necrosis is graded as 1+ and is estimated as 5-10%.

*Day 3 Liver Ribavirin-treated group (Figure 7 i, j) (150X magnification)*

There are widely scattered microfoci of necrosis. The changes are similar in type and extent to those shown in g and h of day 3 Hp-Hb-Ribavirin-treated liver.

Survival

Animals were sacrificed daily post infection to recover tissues for analysis. The number of animals remaining was monitored for survival, and the results as presented include death both from disease and sacrifice. The fraction surviving is calculated based on animals surviving at the beginning of each day at the time of sacrifice, and do not include animals which die by the end of that day. Survival of MHV-3 infected mice treated with hemoglobin-ribavirin exceeded that of free ribavirin despite the fact that the dose of ribavirin in the Hb-conjugate was 1/3 of that of free ribavirin. Results are illustrated in **Figure 8.**

Anti-viral activity *in vivo* and *in vitro*

Livers harvested from MHV-3 infected mice treated with Hp-Hb-RV demonstrated a significantly lower viral titer than mice treated with free ribavirin alone. Results are illustrated in **Figure 9.**

Macrophages treated with Hp-Hb-RV *in vitro* had a marked reduction in MHV-3 viral titers (**Figure 10**) and showed greater inhibition of viral replication (**Figure 11**) in contrast to macrophages treated with free ribavirin alone.

Production of pro-inflammatory mediators including tumor necrosis factor (TNF$\alpha$) and interferon (IFN$\gamma$) were markedly reduced by Hp-Hb-RV as compared with the untreated control. Results are illustrated in **Figure 12** (a) TNF$\alpha$ and (b) IFN$\gamma$.

**TABLE 1 (HRC 203 = Hp-Hb-Ribavirin)**

❖ **HRC 203 treated mice do not develop anemia**

| Group | Day 1 | Day 2 | Day 3 | Day 4 | Day 5 |
|---|---|---|---|---|---|
| **Hematocrit** | | | | | |
| Normal | 41% ± 3% | | | | |
| Control | 41% | N/A | 43% ± 2% | N/A | no animals |
| Ribavirin | 35% | 33% | 33% | N/A | 42% |
| HRC-203 | 38% | 39% | 40% | 40% | 39% |
| **Hemoglobin** | | | | | |
| Normal | 139 ± 12 | | | | |
| Control | 139 | N/A | 147 ± 7 | N/A | no animals |
| Ribavirin | 119 | 112 | 112 | N/A | 143 |
| HRC-203 | 129 | 133 | 136 | 136 | 133 |

## Claims

1. A hemoglobin-nucleoside analogue conjugate for use in treating an inflammatory or viral condition wherein the conjugate directs delivery of the nucleoside analogue to a macrophage, wherein the nucleoside analogue is ribavirin.

2. A hemoglobin-nucleoside analogue conjugate for use according to claim 1 wherein the condition is a viral infection which is selected from the group consisting of Hepatitis C, HIV, SARS, corona virus and RSV.

3. A hemoglobin-nucleoside analogue conjugate for use according to claim 1 or claim 2, which, in use, modulates the immune response and/or the inflammatory response of the macrophage.

4. A hemoglobin-nucleoside analogue conjugate for use according to claim 3, which, in use, modulates the cytokine profile and/or production of the macrophage.

5. A hemoglobin-nucleoside analogue conjugate for use according to claim 4, wherein the cytokines are TNF-$\alpha$ or IFN-$\gamma$.

6. A hemoglobin-nucleoside analogue conjugate for use according to any preceding claim, which is used in conjunction with IFN or PEG-IFN.

7. A hemoglobin-nucleoside analogue conjugate for use according to any preceding claim, wherein the molar ratio of hemoglobin:ribavirin is 1:5-1:20.

8. A hemoglobin-nucleoside analogue conjugate for use according to any preceding claim, which is a hemoglobin-phosphoramidate-ribavirin conjugate.

9. A hemoglobin-nucleoside analogue conjugate for use according to any preceding claim, wherein the hemoglobin is non-intramolecularly cross-linked.

## Patentansprüche

1. Hämoglobin-Nukleosidanalog-Konjugat zur Verwendung bei der Behandlung eines Entzündungs- oder Virusleidens, wobei das Konjugat die Zuführung des Nukleosidanalogs an einen Makrophagen steuert, wobei es sich bei dem Nukleosidanalog um Ribavirin handelt.

2. Hämoglobin-Nukleosidanalog-Konjugat zur Verwendung nach Anspruch 1, wobei es sich bei dem Leiden um eine Virusinfektion handelt, die aus der aus Hepatitis C, HIV, SARS, Coronavirus und RSV bestehenden Gruppe ausgewählt ist.

3. Hämoglobin-Nukleosidanalog-Konjugat zur Verwendung nach Anspruch 1 oder Anspruch 2, das bei seiner Verwendung die Immunantwort und/oder die Entzündungsreaktion des Makrophagen moduliert.

4. Hämoglobin-Nukleosidanalog-Konjugat zur Verwendung nach Anspruch 3, das bei seiner Verwendung das Cytokinprofil und/oder die Cytokinproduktion des Makrophagen moduliert.

5. Hämoglobin-Nukleosidanalog-Konjugat zur Verwendung nach Anspruch 4, wobei es sich bei den Cytokinen um TNF-$\alpha$ oder IFN-$\gamma$ handelt.

6. Hämoglobin-Nukleosidanalog-Konjugat zur Verwendung nach einem vorhergehenden Anspruch, das in Verbindung mit IFN oder PEG-IFN verwendet wird.

7. Hämoglobin-Nukleosidanalog-Konjugat zur Verwendung nach einem vorhergehenden Anspruch, wobei das Hämoglobin:Ribavirin-Molverhältnis 1:5-1:20 beträgt.

8. Hämoglobin-Nukleosidanalog-Konjugat zur Verwendung nach einem vorhergehenden Anspruch, bei dem es sich um ein Hämoglobin-Phosphoramidat-Ribavirin-Konjugat handelt.

9. Hämoglobin-Nukleosidanalog-Konjugat zur Verwendung nach einem vorhergehenden Anspruch, wobei das Hämoglobin nichtintramolekular vernetzt ist.

**Revendications**

1. Conjugué hémoglobine-analogue nucléosidique pour utilisation dans le traitement d'un état pathologique inflammatoire ou viral, où le conjugué dirige la libération de l'analogue nucléosidique dans un macrophage, où l'analogue nucléosidique est la ribavirine.

2. Conjugué hémoglobine-analogue nucléosidique pour utilisation selon la revendication 1, où l'état pathologique est une infection virale qui est choisie dans le groupe constitué par les suivantes : hépatite C, VIH, SRAS, coronavirus et VRS.

3. Conjugué hémoglobine-analogue nucléosidique pour utilisation selon la revendication 1 ou la revendication 2 qui, lors de son utilisation, module la réponse immunitaire et/ou la réponse inflammatoire du macrophage.

4. Conjugué hémoglobine-analogue nucléosidique pour utilisation selon la revendication 3 qui, lors de son utilisation, module le profil et/ou la production de cytokines du macrophage.

5. Conjugué hémoglobine-analogue nucléosidique pour utilisation selon la revendication 4, où les cytokines sont TNF-$\alpha$ ou IFN-$\gamma$.

6. Conjugué hémoglobine-analogue nucléosidique pour utilisation selon l'une quelconque des revendications précédentes, qui est utilisé en conjonction avec IFN ou PEG-IFN.

7. Conjugué hémoglobine-analogue nucléosidique pour utilisation selon l'une quelconque des revendications précédentes, où le rapport molaire hémoglobine:ribavirine est de 1:5-1:20.

8. Conjugué hémoglobine-analogue nucléosidique pour utilisation selon l'une quelconque des revendications précédentes, qui est un conjugué hémoglobine-phosphoramidate-ribavirine.

9. Conjugué hémoglobine-analogue nucléosidique pour utilisation selon l'une quelconque des revendications précédentes, où l'hémoglobine est non réticulée au niveau intramoléculaire.

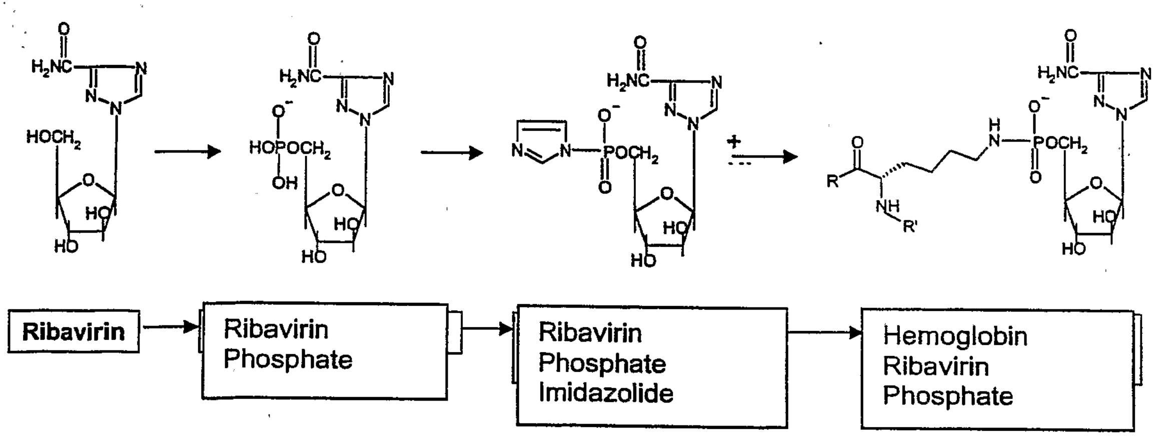
Ribavirin
Ribavirin Phosphate
Ribavirin Phosphate Imidazolide
Hemoglobin Ribavirin Phosphate

**FIGURE 1**

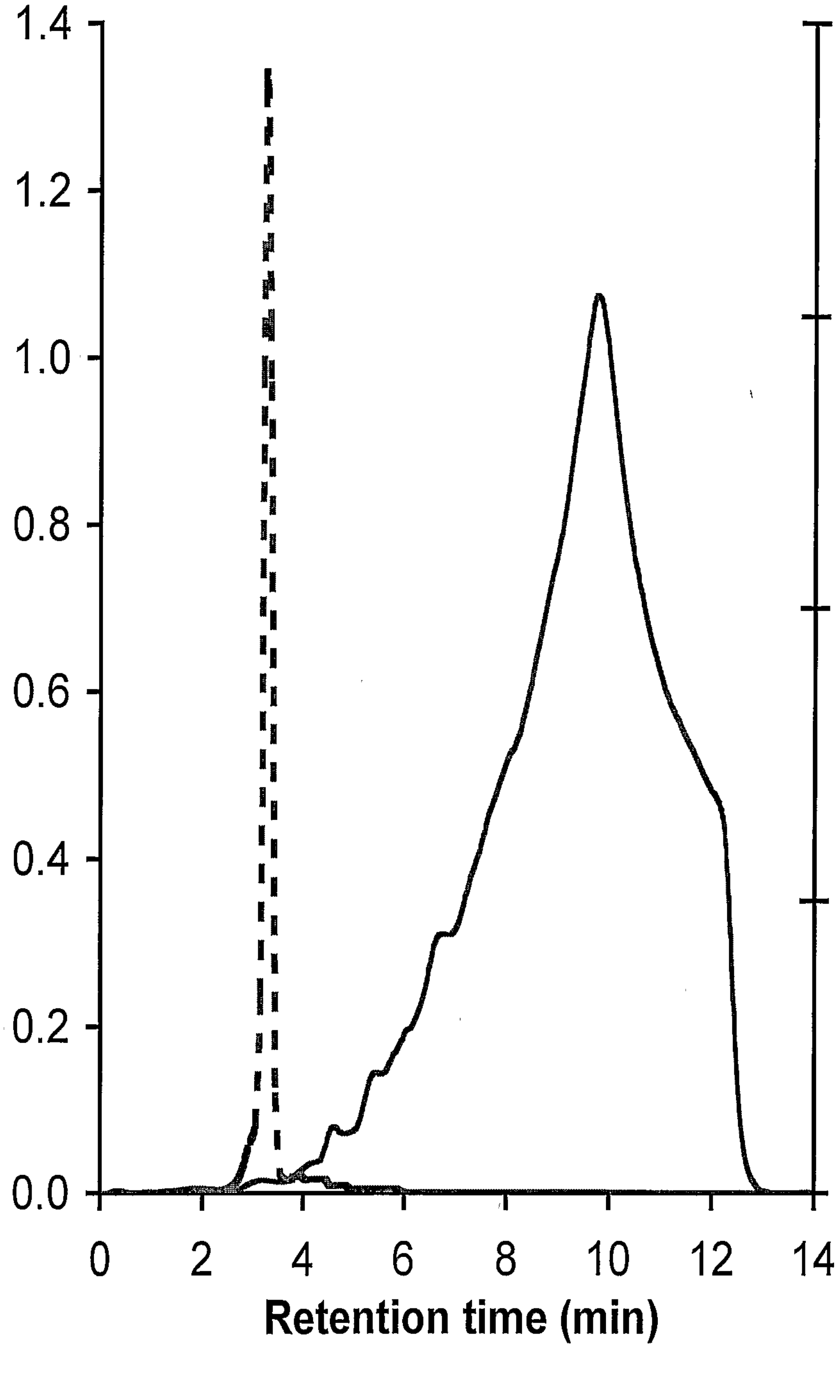


FIGURE 2

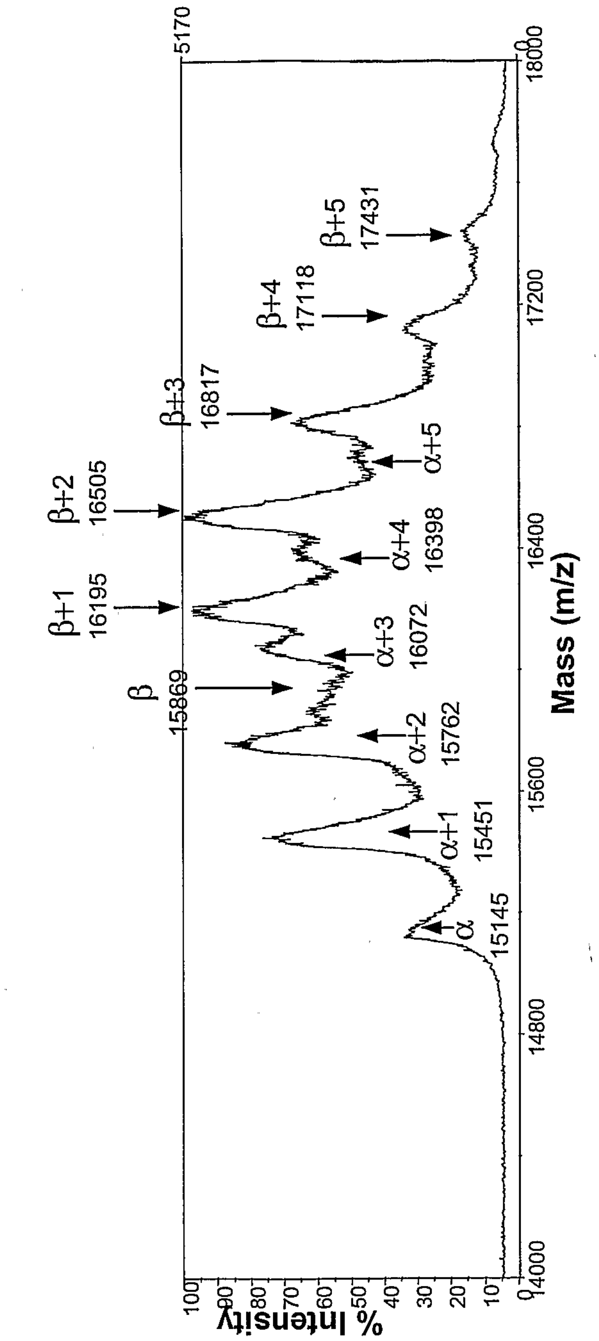

% Intensity
Mass (m/z)
5170
14000
14800
15600
16400
17200
18000
α 15145
α+1 15451
α+2 15762
α+3 16072
α+4 16398
α+5
β 15869
β+1 16195
β+2 16505
β+3 16817
β+4 17118
β+5 17431


FIGURE 3

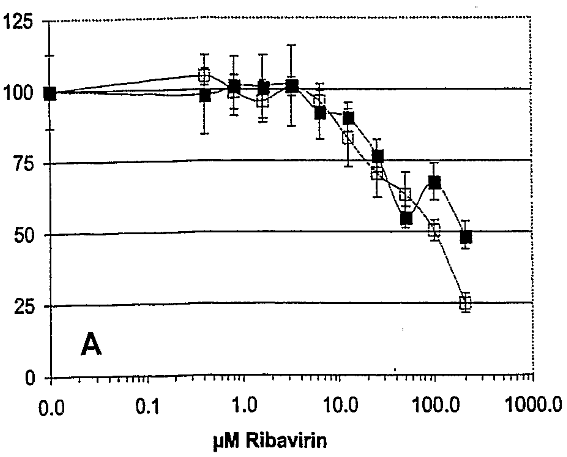
125
100
75
50
25
0
A
0.0
0.1
1.0
10.0
100.0
1000.0
μM Ribavirin

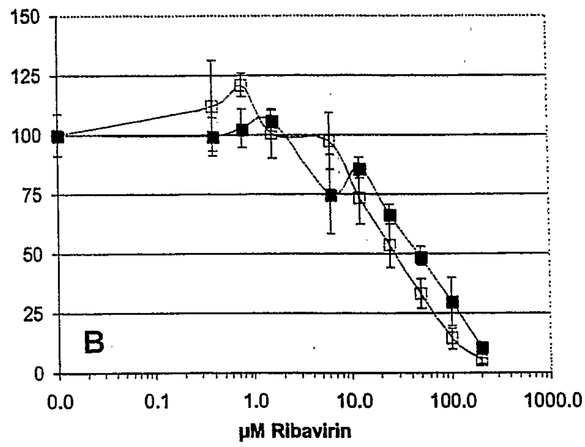
150
125
100
75
50
25
0
B
0.0
0.1
1.0
10.0
100.0
1000.0
μM Ribavirin

FIGURE 4

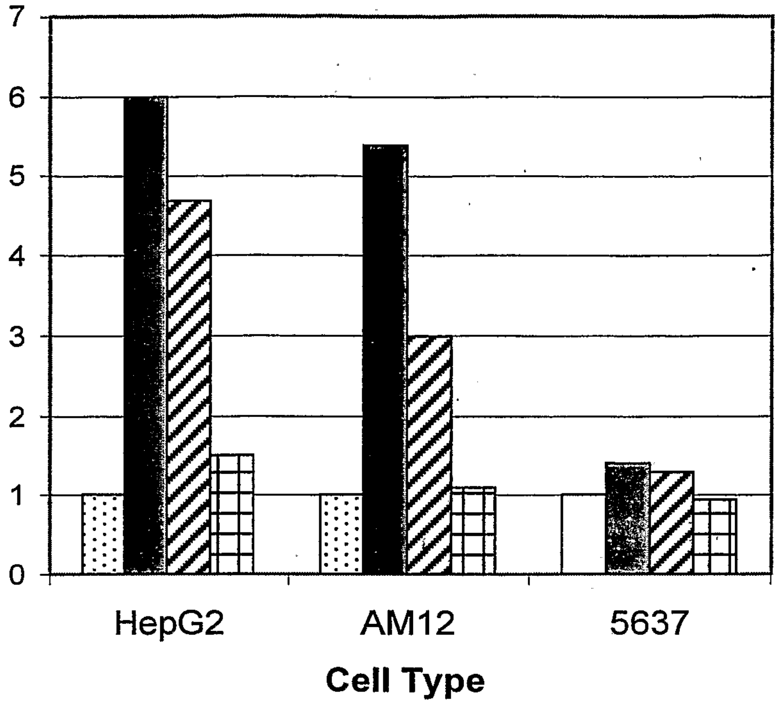


FIGURE 5

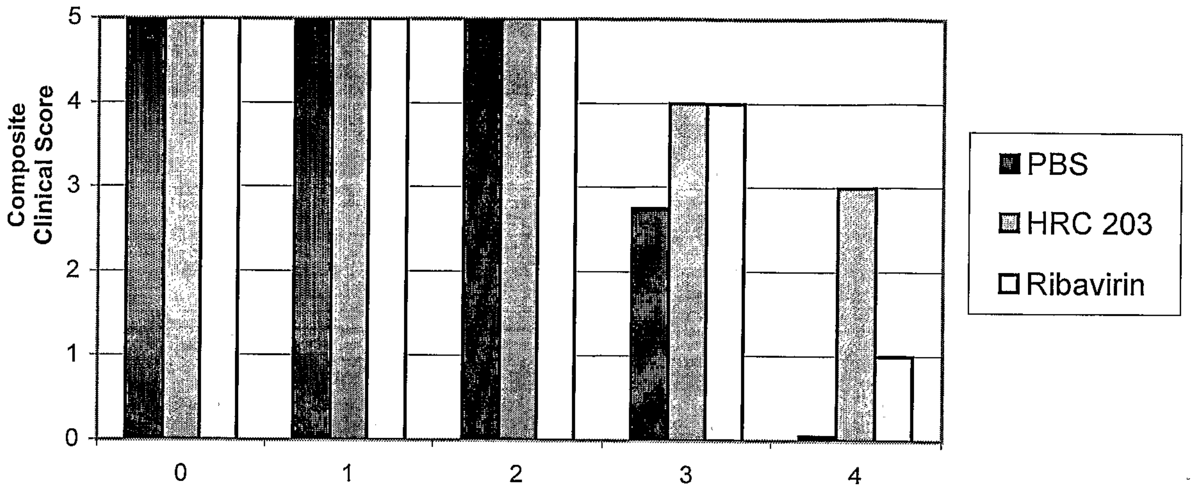
Composite Clinical Score
5
4
3
2
1
0
0
1
2
3
4
PBS
HRC 203
Ribavirin

FIGURE 6

# FIGURE 7

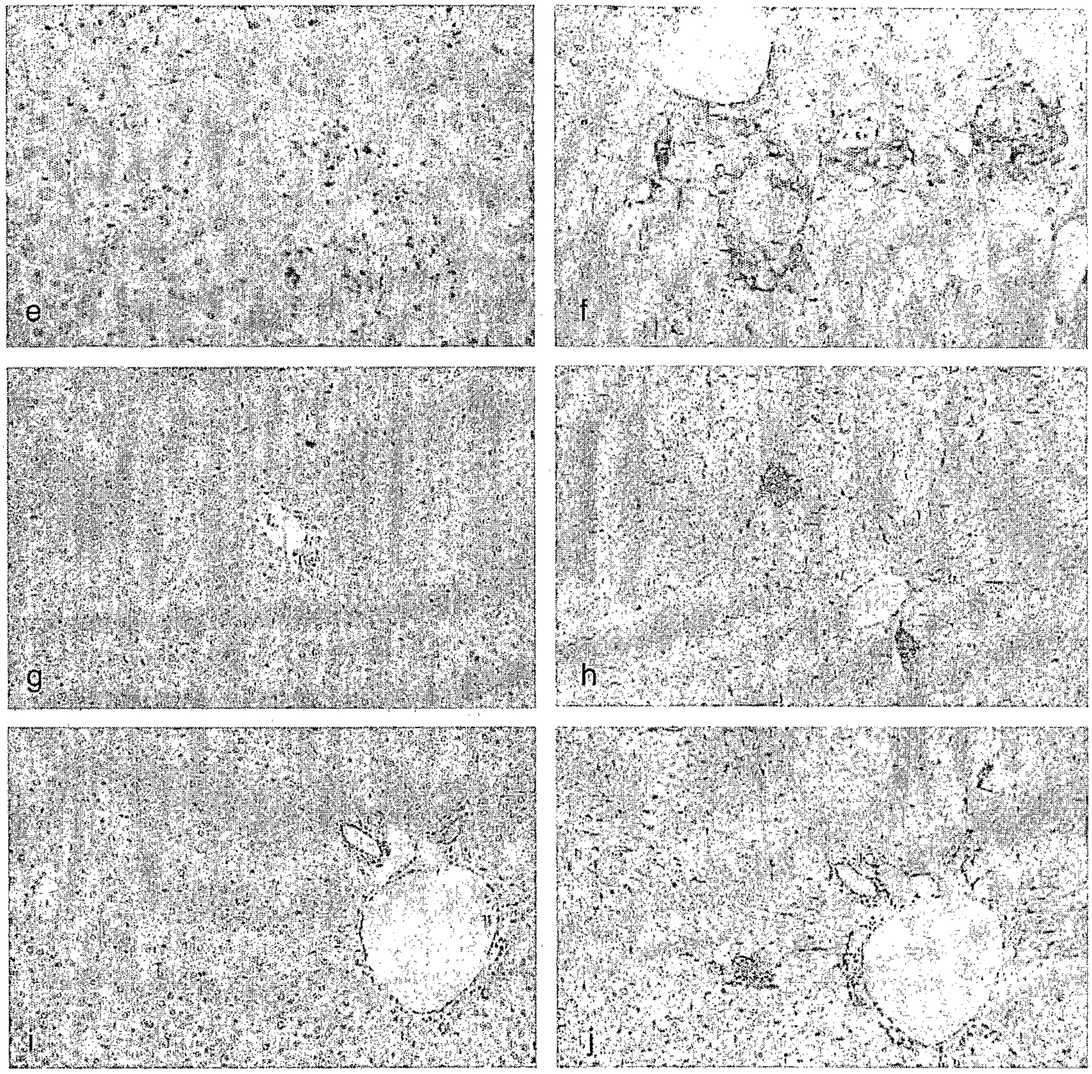

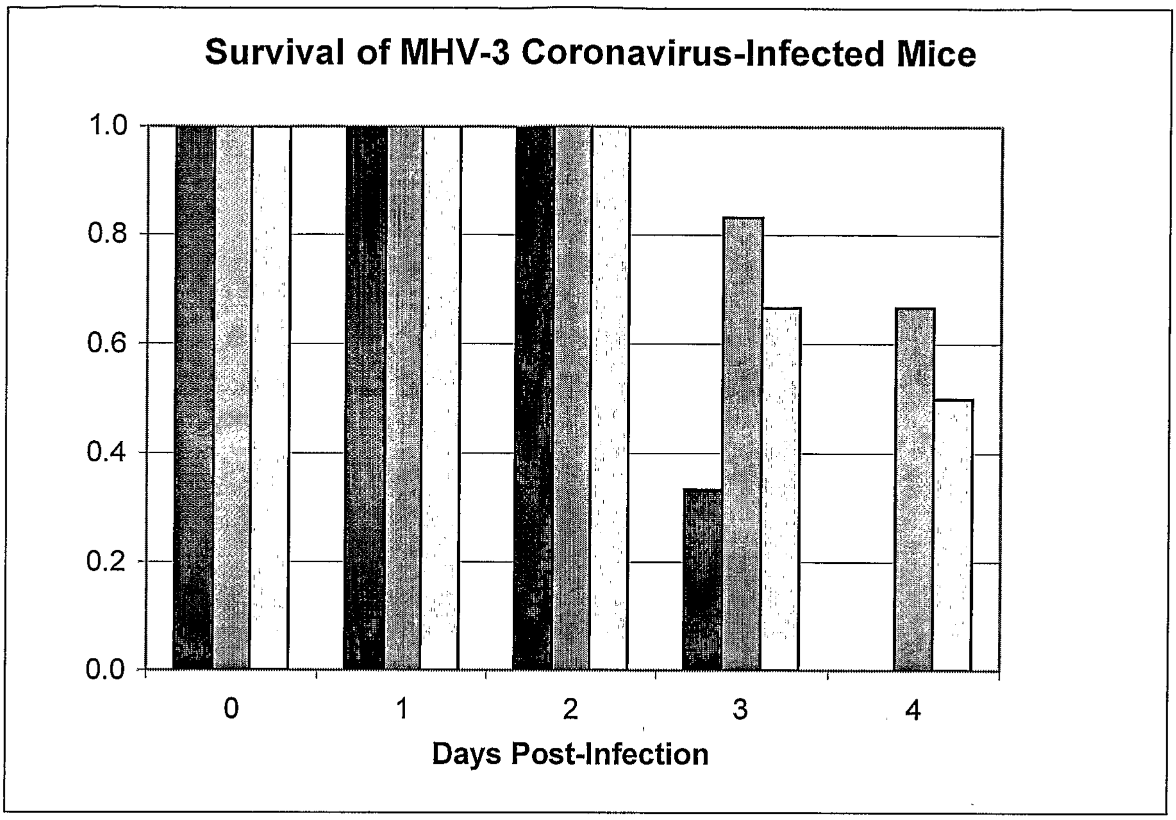
Survival of MHV-3 Coronavirus-Infected Mice
1.0
0.8
0.6
0.4
0.2
0.0
0
1
2
3
4
Days Post-Infection

FIGURE 8

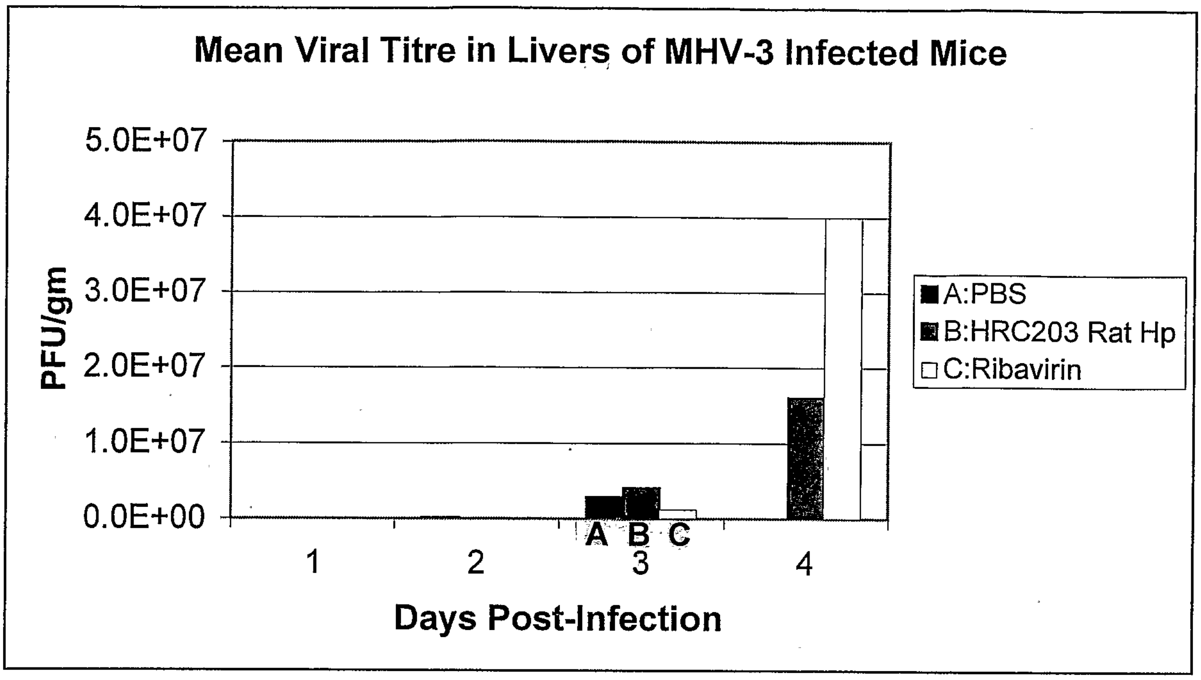
Mean Viral Titre in Livers of MHV-3 Infected Mice
5.0E+07
4.0E+07
3.0E+07
2.0E+07
1.0E+07
0.0E+00
PFU/gm
A B C
1
2
3
4
Days Post-Infection
A:PBS
B:HRC203 Rat Hp
C:Ribavirin

FIGURE 9

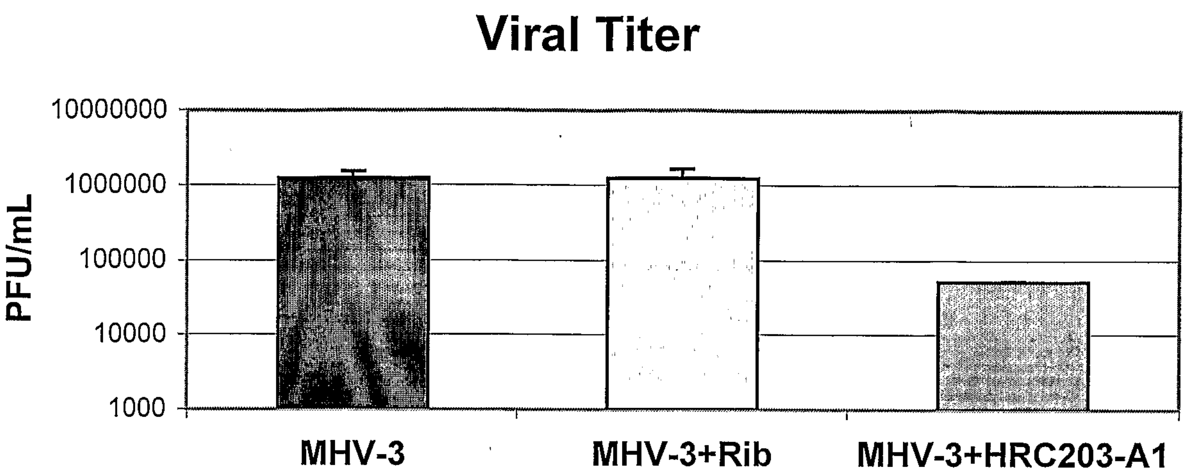
Viral Titer
10000000
1000000
100000
10000
1000
PFU/mL
MHV-3
MHV-3+Rib
MHV-3+HRC203-A1

FIGURE 10

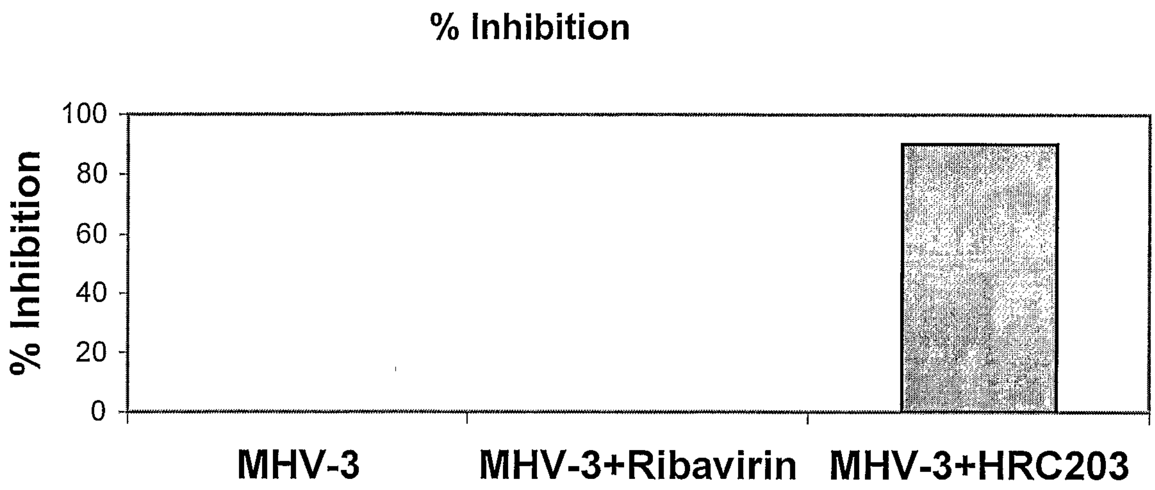
% Inhibition
100
80
60
40
20
0
% Inhibition
MHV-3
MHV-3+Ribavirin
MHV-3+HRC203

FIGURE 11

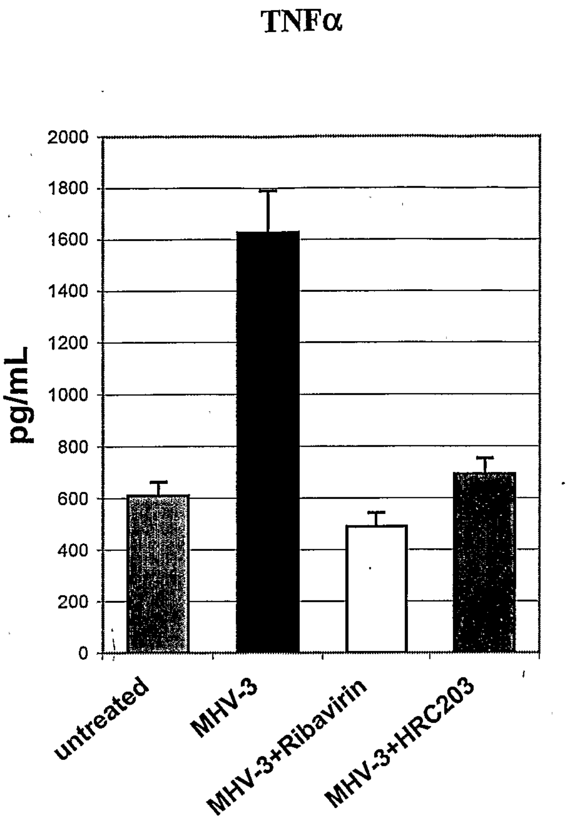


FIGURE 12A

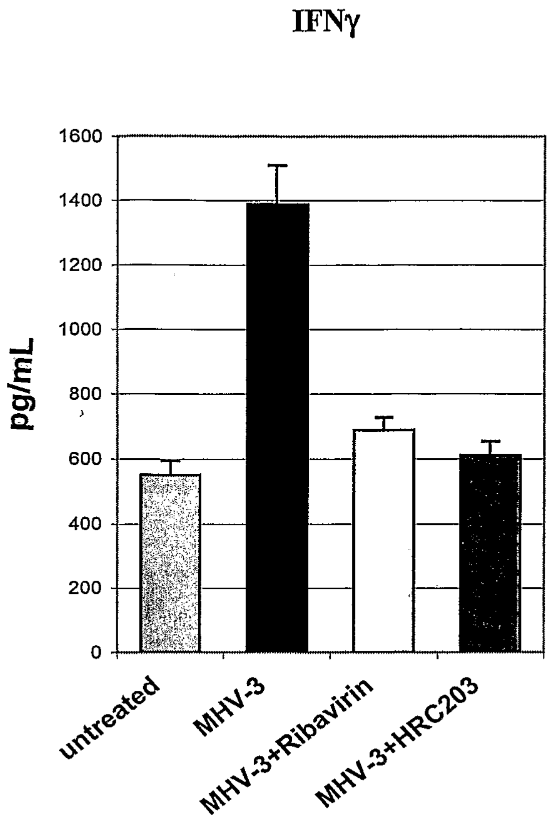


FIGURE 12B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6599887 B
- US 6448932 B
- US 6096311 A
- US 6071517 A
- US 6018031 A
- US 4764359 A
- US 5399 A, Kluger
- US 671 A
- WO 9411399 A
- US 5679777 A, Anderson
- WO 9956723 A
- WO 9218375 A
- US 5439882 A
- WO 9505397 A
- EP 1367393 A
- WO 03100419 A
- US 5439591 A, Pliura
- US 5399671 A, Kluger
- US 6479637 B
- US 5843456 A


### Non-patent literature cited in the description


- **SCHMIDT, J. et al.** *Brain,* 2003, vol. 126 (8), 1895-904
- **KRISTIANSEN et al.** Identification of the haemoglobin scavenger receptor. *Nature,* 2001, vol. 409, 198-201
- **AR KARLSTROM et al.** *Antimicrob Agents Chemother,* 29 January 1986, (1), 171-174
- **HOFFMAN et al.** *Antimicrob. Agents Chemother.,* 1973, vol. 3, 235
- **SIDWELL et al.** *Science,* 1972, vol. 177, 205
- **R. KLUGER et al.** *Artificial Cells, Blood Substitutes, and Immobilization Biotechnology,* 1994, vol. 22 (5), A115
- **S.M. CHAMOW et al.** *Journal of Biological Chemistry,* 1992, vol. 267 (22), 15916-15922
- **E. ANTONINI et al.** *Biochimica Biophysica Acta,* 1964, vol. 2 (82), 355-360
- **H.J. MØLLER et al.** *Blood,* 2002, vol. 99, 378-380
- **G. LEVY et al.** *Hepatology,* 2003, vol. 38 (1), 305A
- **H. LEBLEBICIOGLU et al.** *Annals of Clinical Microbiology and Antimicrobials,* 2002, vol. 1, 3p
- **S. POL et al.** *Journal of Viral Hepatitis,* 2002, vol. 9 (1), 1-8
- **C.O. ZEIN et al.** *Microbes and Infection,* 2002, vol. 4 (12), 1237-1246
- **B.A. LUXON et al.** *Clinical Therapeutics,* 2002, vol. 24 (9), 1363-1383
- **T.J. BROWN et al.** *J Am Acad Dermatol,* 2002, vol. 47, 581-599
- **M. RITTER et al.** *European Journal of Immunology,* 2001, vol. 31 (4), 999-1009
- **M. WILLEMS et al.** *Transplant International,* 2002, vol. 15 (2-3), 61-72
- **KLUGER AND LI.** *Hemoglobin With Chemically Introduced Disulfide Crosslinks and Preparation Thereof,* 03 November 1997
- **KRISTIANSEN et al.** Identification of the haemoglobin scavenger receptor. *Nature,* 11 January 2001, vol. 409, 198-201
- **ALLEN et al.** *J Med Chem.,* August 1978, vol. 21 (8), 742-6
- *Methods Enzymol,* vol. 8, 115-118
- **STREETER et al.** *Proc. Natl. Acad. Sci. USA,* April 1973, vol. 70 (4), 1174-8
- **FIUME et al.** *Anal Biochem.,* 01 August 1993, vol. 212 (2), 407-11
- **ZUWALA-JAGIELLO ; OSADA.** Internalization study using EDTA-prepared hepotocytes for receptor-mediated endocytosis of haemoglobin-haptoglobin complex. *The International Journal of Biochemistry & Cell Biology,* August 1998, vol. 30 (8), 923-931